(19) Europäisches Patentamt / European Patent Office / Office européen des brevets

(11) **EP 3 711 678 B1**

(12) **EUROPEAN PATENT SPECIFICATION**

(45) Date of publication and mention of the grant of the patent:
**14.06.2023 Bulletin 2023/24**

(21) Application number: **20162796.5**

(22) Date of filing: **12.03.2020**

(51) International Patent Classification (IPC):
**A61B 8/08** (2006.01)    **G01S 7/52** (2006.01)

(52) Cooperative Patent Classification (CPC):
**A61B 8/485; G01S 7/52022; G01S 7/52042; G01S 7/52085;** A61B 8/463; A61B 8/469; A61B 8/5246; A61B 8/5276; G01S 7/52095

(54) **ULTRASOUND DIAGNOSTIC APPARATUS AND METHOD OF CONTROLLING THE SAME**

ULTRASCHALLDIAGNOSEVORRICHTUNG UND VERFAHREN ZUR STEUERUNG DAVON

APPAREIL DE DIAGNOSTIC ULTRASONIQUE ET SON PROCÉDÉ DE COMMANDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.03.2019 KR 20190030525**

(43) Date of publication of application:
**23.09.2020 Bulletin 2020/39**

(73) Proprietor: **Samsung Medison Co., Ltd. Hongcheon-gun, Gangwon-do, 25108 (KR)**

(72) Inventors:
• **KONG, Donggeon**
  18431 Gyeonggi-do (KR)
• **LEE, Hyoungki**
  13527 Gyeonggi-do (KR)
• **OH, Yousang**
  44919 Ulsan (KR)

(74) Representative: **Arnold & Siedsma**
**Bezuidenhoutseweg 57**
**2594 AC The Hague (NL)**

(56) References cited:
WO-A1-2018/060820    WO-A1-2018/182308
US-A1- 2013 028 536    US-A1- 2015 192 547
US-A1- 2016 089 113    US-A1- 2017 156 700

• SONG PENGFEI ET AL: "Two-dimensional shear-wave elastography on conventional ultrasound scanners with time-aligned sequential tracking (TAST) and comb-push ultrasound shear elastography (CUSE)", IEEE TRANSACTIONS ON ULTRASONICS, FERROELECTRICS AND FREQUENCY CONTROL, IEEE, US, vol. 62, no. 2, 1 February 2015 (2015-02-01), pages 290-302, XP011571397, ISSN: 0885-3010, DOI: 10.1109/TUFFC.2014.006628 [retrieved on 2015-01-28]
• PENGFEI SONG ET AL: "Fast Shear Compounding Using Robust 2-D Shear Wave Speed Calculation and Multi-directional Filtering", ULTRASOUND IN MEDICINE & BIOLOGY, vol. 40, no. 6, 1 June 2014 (2014-06-01), pages 1343-1355, XP055185962, ISSN: 0301-5629, DOI: 10.1016/j.ultrasmedbio.2013.12.026
• DENG YUFENG ET AL: "Ultrasonic Shear Wave Elasticity Imaging Sequencing and Data Processing Using a Verasonics Research Scanner", IEEE TRANSACTIONS ON ULTRASONICS, FERROELECTRICS AND FREQUENCY CONTROL, IEEE, US , vol. 64, no. 1 12 January 2017 (2017-01-12), pages 164-176, XP011638783, ISSN: 0885-3010, DOI: 10.1109/TUFFC.2016.2614944 Retrieved from the Internet: URL:http://ieeexplore.ieee.org/document/75 81103/ [retrieved on 2016-10-03]

**EP 3 711 678 B1**

**Description**

BACKGROUND

1. Field

The disclosure relates to an ultrasound diagnostic apparatus and a method of controlling the same.

2. Description of the Related Art

Ultrasound diagnostic apparatuses operate to irradiate an ultrasound signal generated from an ultrasound probe transducer to a target site inside an object through the surface of the object and noninvasively acquire tomographic images or blood stream images of soft tissues using information about an ultrasound signal (an ultrasound echo signal) reflected from the object.

The ultrasound diagnostic apparatus has advantages in that it is compact and inexpensive, is displayable in real time, and has high safety compared to X-ray diagnostic devices due to having no risk of exposure to X-rays or the like, and thus are widely used for cardiac, breast, abdominal, urinary, and obstetrical diagnoses.

On the other hand, tissues of a human body have an elasticity, and a lesion tissue may be detected on the basis of the elasticity of the tissue. The ultrasound diagnostic apparatus may measure the elasticity of the tissue and image the elasticity. In detail, the ultrasound diagnostic apparatus may calculate the elasticity by estimating the velocity of a shear wave, and generate an elasticity image of the shear wave.

However, when diagnosing obese patients, severe reverberation occurs due to a fat layer, which causes difficulty in accurately measuring the elasticity.

WO 2018/060820 A1 discloses an ultrasonic diagnostic imaging for analyzing shear wave characteristics that utilizes a background motion compensation subsystem which acts as a spatial filter of pulse-to-pulse autocorrelation phases over the ROI of tracking pulse vectors to compensate for background motion. The subsystem is configured to compute the sum of all lag-1 autocorrelations of tracking line ensemble data over the tracking ROI, for each PRI.

Song Pengfei et al., "Two-dimensional shear-wave elastography on conventional ultrasound scanners with time-aligned sequential tracking (TAST) and comb-push ultrasound shear elastography (CUSE)", in IEEE TRANSACTIONS ON ULTRASONICS, FERROELECTRICS AND FREQUENCY CONTROL, IEEE, US, vol. 62, no. 2 (1 February 2015) discloses a time-aligned sequential tracking (TAST) method for shear-wave tracking on conventional ultrasound scanners. TAST takes advantage of the parallel beamforming capability of conventional systems and realizes high-PRF shear-wave tracking by sequentially firing tracking vectors and aligning shear wave data in the temporal direction. The comb-push ultrasound shear elastography (CUSE) technique was used to simultaneously produce multiple shear wave sources within the field-of-view (FOV) to enhance shear wave SNR and facilitate robust reconstructions of 2D elasticity maps.

Song Pengfei et al., "Fast Shear Compounding Using Robust 2-D Shear Wave Speed Calculation and Multidirectional Filtering", in ULTRASOUND IN MEDICINE & BIOLOGY, vol. 40, no. 6 (1 June 2014) discloses a fast shear compounding method using only one shear wave push-detect cycle, such that the shear wave imaging frame rate is preserved and motion artifacts are minimized. The proposed method is composed of the following steps: applying a comb-push to produce multiple differently angled shear waves at different spatial locations simultaneously; decomposing the complex shear wave field into individual shear wave fields with differently oriented shear waves using a multidirectional filter; using a robust 2D shear wave speed calculation to reconstruct 2D shear elasticity maps from each filter direction; and compounding these 2D maps from different directions into a final map.

SUMMARY

The invention is defined in the independent claims.

Therefore, it is an object of the disclosure to provide an ultrasound diagnostic apparatus capable of accurately measuring the elasticity even in the presence of reverberation by improving the performance of shear wave observation, and a method of controlling the same.

It is another object of the disclosure to provide an ultrasound diagnostic apparatus capable of accurately measuring the elasticity by setting the interval of tracking pulses for shear wave observation to be wide when a region of interest (ROI) is set wide, and a method of controlling the same.

Additional aspects of the disclosure will be set forth in part in the description which follows and, in part, will be obvious from the description, or may be learned by practice of the disclosure.

Therefore, it is an aspect of the disclosure to provide a method of controlling an ultrasound diagnostic apparatus, according to claim 1.

The method may further include setting the ROI in a radial form, wherein the transmitting of the plurality of tracking pulses includes radially transmitting the plurality of tracking pulses to the ROI in the radial from.

The adjusting of the position of the focal point may include moving the focal point into the ROI in response to movement of the ROI.

The receiving of the ultrasound echo signals includes setting sets of multi-reception scan lines, each set corresponding to a respective one of the plurality of tracking pulses, wherein the estimating of the velocity of the shear wave may include selectively performing signal processing on the multi-reception scan lines.

The estimating of the velocity of the shear wave may include: grouping reception scan lines positioned at a same relative position in each of the sets of the multi-reception scan lines to generate a plurality of groups; estimating a plurality of velocities of the shear wave each corresponding to a respective one of the plurality of groups; and determining a final velocity of the shear wave on the basis of the plurality of the velocities of the shear wave.

The determining of the final velocity of the shear wave may include determining an average value of the plurality of velocities of the shear wave or a weighted average value obtained using a reliability measurement index (RMI) on each of the plurality of velocities of the shear wave as the final shear wave.

The estimating of the velocity of the shear wave may include: selecting some reception scan lines from the multi-reception scan lines; and estimating the velocity of the shear wave on the basis of ultrasound echo signals received along the selected some reception scan lines.

The selecting of the reception scan lines may include selecting reception scan lines adjacent to each of the plurality of tracking pulses from the sets of the multi-reception scan lines.

The selecting of the reception scan lines may include selecting reception scan lines having a positional error smaller than a predetermined value.

The estimating of the velocity of the shear wave may further include estimating an arrival time of the shear wave on each of the multi-reception scan lines, wherein the selecting of the reception scan lines may include selecting reception scan lines except for a reception scan line in which the shear wave has a minimum arrival time and a reception scan line in which the shear wave has a maximum arrival time.

The outputting of the shear wave elasticity image may include displaying an elasticity, a depth, and a reliability measurement index (RMI).

The transmitting of the plurality of tracking pulses may include transmitting the plurality of tracking pulses in an interleaving method.

The estimating of the velocity of the shear wave may include: detecting a displacement of a tissue at a plurality of sampling points of each of the multi-reception scan lines; estimating an arrival time of the shear wave on each of the multi-reception scan lines on the basis of the displacement of the tissue; and estimating the velocity of the shear wave on the basis of a distance between the multi-reception scan lines and a difference between the arrival times of the shear wave on the multi-reception scan lines.

It is another aspect of the disclosure to provide an ultrasound diagnosis apparatus according to claim 9.

The controller may set the ROI in a radial form, and control the ultrasound probe to radially transmitting the plurality of tracking pulses to the ROI in the radial from.

The controller may move the focal point into the ROI in response to movement of the ROI.

The controller arranges sets of multi-reception scan lines, each set corresponding to a respective one of the plurality of tracking pulses, and may selectively perform signal processing on the multi-reception scan lines.

The controller may group reception scan lines positioned at a same relative position in each of the sets of the multi-reception scan lines to generate a plurality of groups, may estimate a plurality of velocities of the shear wave each corresponding to a respective one of the plurality of groups, and may determine a final velocity of the shear wave on the basis of the plurality of the velocities of the shear wave.

The controller may determine an average value of the plurality of velocities of the shear wave or a weighted average value obtained using a reliability measurement index (RMI) on each of the plurality of velocities of the shear wave as the final shear wave.

The controller may select some reception scan lines from the multi-reception scan lines, and estimate the velocity of the shear wave on the basis of ultrasound echo signals received along the selected some reception scan lines.

The controller may select reception scan lines adjacent to each of the plurality of tracking pulses from the sets of the multi-reception scan lines.

The controller may select reception scan lines having a positional error smaller than a predetermined value.

The controller may estimate an arrival time of the shear wave on each of the multi-reception scan lines, and select reception scan lines except for a reception scan line in which the shear wave has a minimum arrival time and a reception scan line in which the shear wave has a maximum arrival time.

The controller may control the display to display an elasticity, a depth, and a reliability measurement index (RMI).

The controller may control the ultrasound probe to transmit the plurality of tracking pulses in an interleaving method.

The controller may detect a displacement of a tissue at a plurality of sampling points of each of the multi-reception scan lines, estimate an arrival time of the shear wave on each of the multi-reception scan lines on the basis of the displacement of the tissue, and estimate the velocity of the shear wave on the basis of a distance between the multi-reception scan lines and a difference between the arrival times of the shear wave on the multi-reception scan lines.

BRIEF DESCRIPTION OF THE DRAWINGS

These and/or other aspects of the disclosure will become apparent and more readily appreciated from the following description of the embodiments, taken in conjunction with the accompanying drawings of which:

FIG. 1 illustrates an ultrasound diagnostic apparatus according to an embodiment.
FIG. 2 is a block diagram illustrating a configuration of an ultrasound diagnostic apparatus according to an embodiment.
FIG. 3 is a block diagram illustrating a configuration of an ultrasound probe according to an embodiment.
FIG. 4 illustrates transmission and reception of ultrasound waves.
FIG. 5 is a flowchart schematically showing a method of estimating the shear wave velocity.
FIG. 6 illustrates induction of a shear wave by a push pulse.
FIG. 7 illustrates propagation of a shear wave.
FIG. 8 illustrates an example of a method of detecting a shear wave.
FIG. 9 illustrates transmission of a tracking pulse according to another example of a method of detecting a shear wave.
FIG. 10 illustrates a method of transmitting a plurality of tracking pulses to widen an observing area.
FIG. 11 illustrates radial transmission of a plurality of tracking pulses to suit a region of interest.
FIG. 12 illustrates multi-reception scan lines corresponding to a single tracking pulse.
FIG. 13 illustrates a plurality of tracking pulses and a sequence of sets of multi-reception scan lines.
FIG. 14 illustrates the relationship between the displacement of a tissue and the arrival time of a shear wave.
FIG. 15 illustrates the positional error of multi-reception scan lines.
FIG. 16 illustrates the error of the shear wave arrival time on each of the multi-reception scan lines.
FIG. 17 illustrates reception scan lines positioned at the same relative location in the sets of the multi-reception scan lines.
FIG. 18 illustrates the shear wave arrival times for reception scan lines positioned at the same relative location.
FIG. 19 is a flowchart showing a method of controlling an ultrasound diagnostic apparatus, which describes a method of estimating the shear wave velocity by grouping reception scan lines.
FIG. 20 illustrates a wave front graph for describing the method of estimating the shear wave velocity shown in FIG. 19.
FIG. 21 is a flowchart showing a method of controlling an ultrasound diagnostic apparatus according to another embodiment, which describes a method of estimating the shear wave velocity by selecting some reception scan lines.
FIGS. 22 and 23 show wave front graphs for describing the method of estimating the shear wave velocity shown in FIG. 21.
FIGS. 24 and 25 illustrate the intervals between a plurality of tracking pulses.
FIGS. 26 and 27 show the result of the elasticity measurement according to the related art.
FIG. 28 shows the result of the elasticity measurement by the method of controlling the ultrasound diagnostic apparatus according to the embodiment.

DETAILED DESCRIPTION

Like numerals refer to like elements throughout the specification. Not all elements of embodiments of the present disclosure will be described, and description of what are commonly known in the art or what overlap each other in the embodiments will be omitted.

It will be further understood that the term "connect" or its derivatives refer both to direct and indirect connection, and the indirect connection includes a connection over a wireless communication network.

It will be further understood that the terms "comprises" and/or "comprising," when used in this specification, specify the presence of stated features, integers, steps, operations, elements, and/or components, but do not preclude the presence or addition of one or more other features, integers, steps, operations, elements,

Although the terms "first," "second," "A," "B," etc. may be used to describe various components, the terms do not limit the corresponding components, but are used only for the purpose of distinguishing one component from another component. As used herein, the singular forms "a," "an" and "the" are intended to include the plural forms as well, unless the context clearly indicates otherwise.

Moreover, terms described in the specification such as "part," "module," and "unit," refer to a unit of processing at least one function or operation, and may be implemented by software, a hardware component such as a field-programmable gate array (FPGA) or an application-specific integrated circuit (ASIC), or a combination of software and hardware.

Reference numerals used for method steps are just used for convenience of explanation, but not to limit an order of the steps. Thus, unless the context clearly dictates otherwise, the written order may be practiced otherwise.

An 'object' may include a person or animal, or part of a person or animal. For example, the object may include not only a mass but also organs such as the liver, heart, uterus, brain, breast, abdomen, or blood vessels. In addition, in the specification, the "user" may be a doctor, a nurse, a clinical pathologist, a medical imaging expert, or the like, and may be a technician who develops and repairs a medical device, but is not limited thereto.

The term "ultrasound image" and "image of an object" refer to an image of an object obtained using ultrasound waves.

Hereinafter, embodiments of the disclosure will be described in detail with reference to the accompanying drawings

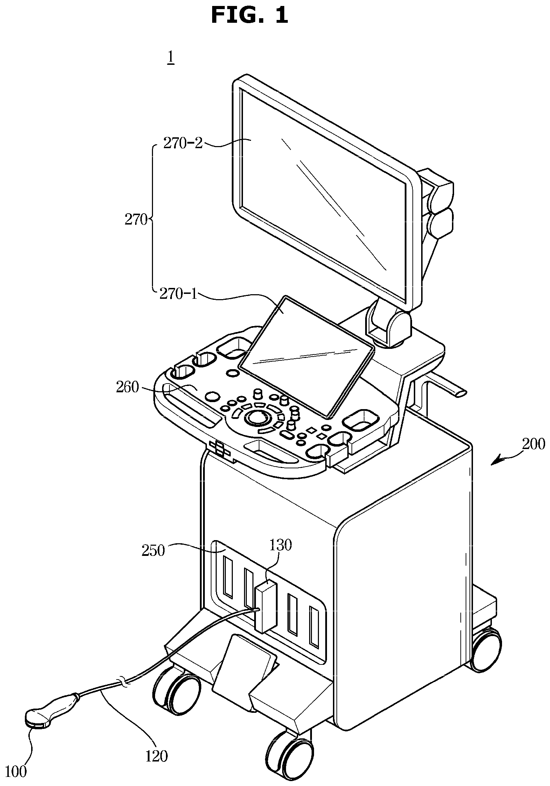

FIG. 1 illustrates an ultrasound diagnostic apparatus according to an embodiment.

Referring to FIG. 1, the ultrasound diagnostic apparatus 1 includes an ultrasound probe 100 and a main body 200. The ultrasound probe 100 may transmit an ultrasound signal to an object to be diagnosed and receive an ultrasound echo signal reflected from the object. The ultrasound probe 100 receives the ultrasound echo signal reflected from the object and converts the ultrasound echo signal into an electrical signal (hereinafter, referred to as an ultrasound signal).

The ultrasound probe 100 may be connected to the main body 200 of the ultrasound diagnostic apparatus 1 through a cable 120, and may receive various signals required for controlling the ultrasound probe P from the main body 200. In addition, the ultrasound probe 100 may transmit an analog signal or a digital signal corresponding to the ultrasound echo signal to the main body 200.

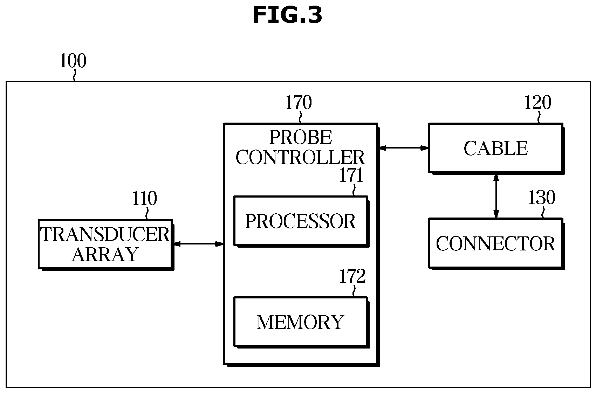

Meanwhile, the ultrasound probe 100 may be implemented as a wireless probe, and may transmit and receive a signal through a network formed between the probe 100 and the main body 200. A detailed description of the probe 100 is described below with reference to FIG. 3.

The main body 200 may include a probe select assembly (PSA) board 250, a control panel 260, and a display 280 (280-1 and 280-2). The PSA board 250 includes a port connected to the ultrasound probe 100. The PSA board 250 may activate the ultrasound probe 100 according to a user command input through the control panel 260 and the control of the controller 300. One end of the cable 120 includes a connector 130 connectable to the port of the PSA board 250.

The control panel 260 is a device that receives a command for operating the ultrasound diagnostic apparatus 1 from a user. The control panel 260 may receive setting information regarding the probe 100, and receive various control commands related to the operation of the main body 200.

The control panel 260 may include a keyboard. The keyboard may include buttons, switches, knobs, touch pads, trackballs, and the like. In addition, the control panel 260 may include a first display 270-1. The first display 270-1 may display a graphic user interface (GUI) for controlling the operation of the ultrasound diagnostic apparatus 1. The first display 270-1 may display related information, such as a menu or an auxiliary image, for optimizing the ultrasound image.

The first display 270-1 may include a touch panel and receive a user's touch input on the graphic user interface. The first display 270-1 may display a graphic user interface having the same shape as a button included in a keyboard. The user may input a command for controlling the ultrasound diagnostic apparatus 1 through a touch input to the first display 270-1.

The second display 270-2 may display an ultrasound image. The ultrasound image may be a two-dimensional (2D) ultrasound image or a three dimension (3D) stereoscopic ultrasound image, and various ultrasound images may be displayed according to an operation mode of the ultrasound diagnostic apparatus 1. In addition, the second display 270-2 may display menus, guide items, information about an operation state of the probe 100, and the like, which are required for the ultrasound diagnosis.

The second display 270-2 may display a shear wave elasticity image that overlaps or is registered with a reference ultrasound image.

The second display 270-2 may also include a touch panel and receive a user's touch input on the graphic user interface. The user may input a command for controlling the ultrasound diagnostic apparatus 1 through a touch input on the second display 270-2.

The display 270 may be implemented as various display devices, such as a liquid crystal display (LCD), a light emitting diode (LED), a plasma display panel (PDP), and an organic light emitting diode (OLED).

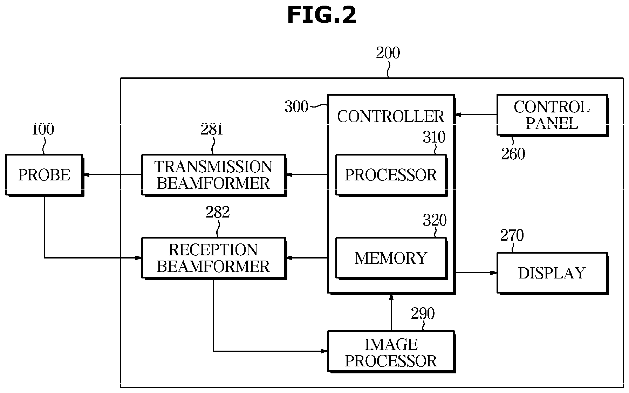

FIG. 2 is a block diagram illustrating a configuration of an ultrasound diagnostic apparatus according to an embodiment.

Referring to FIG. 2, the ultrasound probe 100 may be a linear array probe, a curved array probe, a phased array probe, or a volume probe. The ultrasound probe 100 is not limited thereto, and may include various probes, such as an endocavity probe, a convex probe, a matrix probe, and/or a 3D probe.

The main body 200 of the ultrasound diagnostic apparatus 1 may further include beamformers 281 and 282, an image processor 290, and a controller 300.

The beamformer may be divided into a transmission beamformer 281 and a reception beamformer 282. In obtaining an image using an ultrasound signal, a beamforming technique is used to increase the resolution of the image. The transmission beamformer 281 may apply a transmission pulse to the ultrasound probe 100. The transmission beamformer 281 may apply an appropriate time delay so that ultrasound signals to be transmitted by a plurality of transducer elements are simultaneously focused at one focal point, and generate a transmission beam. A transducer array 110 may transmit the transmission beam to a target site in the object.

In addition, the transmission beamformer 281 may generate a push pulse transmitted along a push line. The push pulse may be irradiated to a region of interest (ROI) R of an object to induce displacement of a tissue and induce shear waves. The displacement of the tissue is used to measure the shear wave velocity, which will be described below. The push pulse may be a focused beam with a relatively high focusing.

The ultrasound transmitted to the object may be reflected from the object and may be incident back to the transducer array 110 of the ultrasound probe 100. The reflected ultrasound signal may be defined as an ultrasound echo signal.

The reception beamformer 281 performs analog/digital conversion on the ultrasound echo signal received from the ultrasound probe 100 and performs reception beamforming. The reception beamformer 281 may apply a time delay to the ultrasound echo signals reflected from the focal point and returning to the transducer elements and add up the ultrasound echo signals at the same time.

Meanwhile, the beamformers 281 and 282 may be provided in the ultrasound probe 100. For example, when the ultrasound probe 100 is a wireless probe, the ultrasound probe 100 may include beamformers 281 and 282.

The image processor 290 filters out noise components in a reception beam to improve the image quality of the ultrasound image, performs an envelope detection process for detecting the intensity of the received signal, and generates digital ultrasound image data.

The image processor 290 may perform scan conversion to convert scan lines of the digital ultrasound image data such that the digital ultrasound image data is displayed on the display 270. In addition, the image processor 290 performs image processing on the ultrasound echo signal to generate an A-mode image, a B-mode image, a D-mode image, an E-mode image, an M-mode image, a Doppler image, and/or a 3D ultrasound image. The image processor 290 performs RGB-processing on the ultrasound image data such that the ultrasound image is displayed on the display 270 and transmits the ultrasound image data to the display 270.

In addition, the image processor 290 may perform image processing for displaying various pieces of additional information on the ultrasound image.

Although the image processor 290 is illustrated as being separated from the controller 300 in FIG. 2, the controller 300 may include the image processor 290.

The display 270 may display the ultrasound image and various types of information processed by the ultrasound diagnostic apparatus 1. The display 270 may display various graphic user interfaces for adjusting the generated ultrasound image.

The controller 300 may control the operation of the ultrasound diagnostic apparatus 1 and the signal flow between internal components of the ultrasound diagnostic apparatus 100. The controller 300 may include a processor 310 and a memory 320. The controller 300 may be implemented as a processing board in which the processor 310 and the memory 320 are installed on a circuit board. The processor 310 and the memory 320 may be connected through a bus. The processor 310 may be provided in a single unit or in a plurality of units thereof.

The controller 300 may be implemented with a plurality of logic gates or a combination of a general-purpose microprocessor and a memory 320 configured to store a program that may be executed in the microprocessor.

The memory 320 refers to a storage medium that stores algorithms and data required for the operation of each component of the ultrasound diagnostic apparatus 1. The memory 320 may include high-speed random-access memory, a magnetic disk, an SRAM, a DRAM, a ROM, or the like. In addition, the memory 320 may be detachable from the ultrasound diagnostic apparatus 1. The memory 320 may include a compact flash (CF) card, a secure digital (SD) card, a smart media (SM) card, a multimedia card (MMC), or a memory stick, but is not limited thereto.

The controller 300 may be electrically connected to each of the PSA board 250, the control panel 260, the display 270, and the beamformers 281 and 282, and may generate a control signal to control components of the probe 100 and the main body 200.

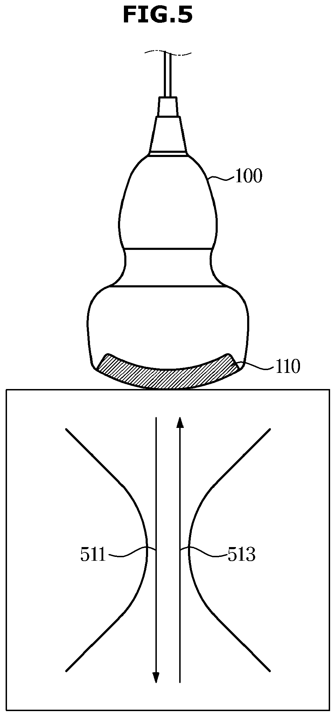

A detailed operation of the controller 300 is described below with reference to FIGS. 5 to 14.

FIG. 3 is a block diagram illustrating a configuration of an ultrasound probe according to an embodiment.

Referring to FIG. 3, the ultrasound probe 100 may include a transducer array 110, a cable 120, a connector 130, and a probe controller 170.

he transducer array 110 is provided at an end of the ultrasound probe 100. The transducer array 110 includes an array of a plurality of ultrasound transducer elements. The transducer array 110 generates ultrasound waves while vibrating by a pulse signal or an alternating current applied by the transmission beamformer 281 of the main body 200. The generated ultrasound is transmitted to a target site inside an object.

The ultrasound generated by the transducer array 110 may be transmitted to a plurality of focuses for a plurality of target sites inside the object. That is, the ultrasound may be multi-focused and transmitted to the plurality of target sites.

The ultrasound transmitted by the transducer array 110 returns to the transducer array 110 as an ultrasound echo signal reflected from the target site inside the object. Upon arrival of the ultrasound echo signal, the transducer array 110 vibrates at a predetermined frequency corresponding to the frequency of the ultrasound echo signal and outputs an alternating current having a frequency corresponding to the vibration frequency. Accordingly, the transducer array 110 may convert the ultrasound echo signal into a predetermined electrical signal.

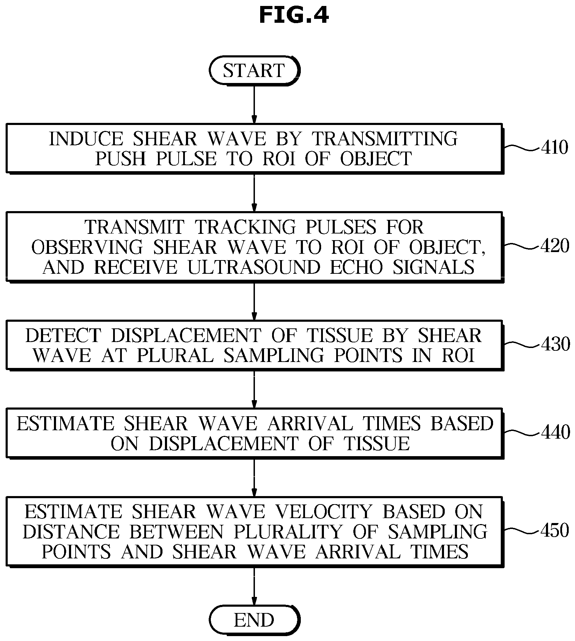

Referring to FIG. 4, the ultrasound probe 100 may transmit a reference pulse 511 to an ROI and receive a first ultrasound echo signal 513 as a result of the reference pulse 511 being reflected from the region of interest. The reference pulse 511 has a beam profile. The width of the beam profile may be adjusted.

The ultrasound diagnostic apparatus 1 may generate a first ultrasound image on the basis of the first ultrasound echo signal 513. The first ultrasound image may be a reference ultrasound image distinguished from a shear wave elasticity image, that represents the position of a tissue before a force is applied to the ROI. The first ultrasound image may be a B-mode image or an M-mode image of the ROI.

Meanwhile, the ultrasound probe 100 may induce a shear wave by transmitting a push pulse to an ROI of the object, and may transmit a tracking pulse for observing the shear wave to the ROI of the object and receive an ultrasound echo signal as a result of reflection of the tracking pulse. The ultrasound echo signal obtained by reflection of the tracking pulse may be defined as a second ultrasound echo signal. The controller 300 of the ultrasound diagnostic apparatus 1 may generate a second ultrasound image on the basis of the second ultrasound echo signal. That is, the second ultrasound echo signal may be defined as a shear wave photographing image.

The transducer elements included in the transducer array 110 may be selectively activated. By selective activation of the transducer elements, the width of the transmission beam may be adjusted. In addition, the plurality of tracking pulses may be transmitted at a preset interval.

The probe controller 170 may include a processor 171 and a memory 172. The processor 171 of the probe controller 170 may be a general micro-processor, and the memory 172 may store a program that may be executed by the processor 171. The probe controller 170 transmits and receives data into and from the main body 200 and controls the overall operation of the probe 100.

The ultrasound probe 100 may further include a T/R switch and a beamformer. The T/R switch serves as a switch to control the conversion between an operation of the transducer array 110 irradiating the ultrasound signal and an operation of the transducer array 110 receiving the reflected echo signal.

Components included in the probe 100 are not limited to those shown in FIG. 3, and may be provided in various combinations.

FIG. 4 is a flowchart schematically showing a method of estimating the shear wave velocity.

Referring to FIG. 4, the ultrasound probe 100 may induce a shear wave by transmitting a push pulse to an ROI of an object (410). In detail, the ultrasound probe 100 may induce displacement in a tissue in the object by irradiating a focused beam to the object. When the focused beam is irradiated to the object, deformation occurs according to an axial movement of the tissue in the object by the focused beam, so that displacement of the tissue is induced. The tissue displacement may cause a shear wave to be propagated.

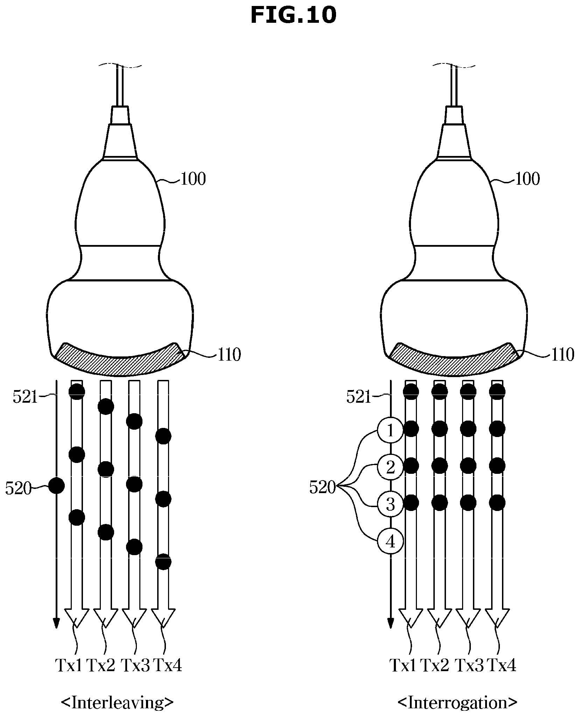

Thereafter, the ultrasound probe 100 may transmit tracking pulses for observing the shear wave to the ROI of the object, and receive ultrasound echo signals a result of reflection of the tracking pulses (420). The ultrasound echo signal resulting from reflection of the tracking pulse may be defined as a second ultrasound echo signal. The tracking pulse has a beam profile of a predetermined width. The tracking pulses may be sequentially transmitted to a plurality of points multi-times. Such a shear wave observation method is referred to as an interleaving method. This will be described in detail with reference to FIG. 10.

Meanwhile, the controller 300 may generate a second ultrasound image on the basis of the second ultrasound echo signal. That is, the second ultrasound echo signal may be defined as a shear wave photographing image.

The controller 300 of the ultrasound diagnostic apparatus 1 may detect the displacement of the tissue at a plurality of sampling points in the ROI (430). Specifically, the controller 300 may set a plurality of transmission scan lines such that a plurality of tracking pulses are transmitted to different positions, and detect the displacement of the tissue at a plurality of sampling points corresponding to the plurality of transmission scan lines. For example, the controller 300 may detect displacement of a tissue by performing cross correlation on the first ultrasound image that is a reference ultrasound image and the second ultrasound image that is a shear wave image.

The controller 300 may estimate the time at which the shear wave arrives at the tissue on the basis of the displacement of the tissue (440). In detail, the controller 300 may estimate the point in time at which the displacement of the tissue is the maximum as the shear wave arrival time. The controller 300 may estimate the shear wave arrival time on each of the plurality of sampling points.

The controller 300 may estimate the shear wave velocity on the basis of the distance between the plurality of sampling points and the shear wave arrival times (450). For example, the controller 300 may calculate the shear wave velocity using a distance between two sampling points located in the traveling direction of the shear wave and the shear wave arrival times for the two sampling points.

Hereinafter, a method of estimating the shear wave velocity will be described in more detail.

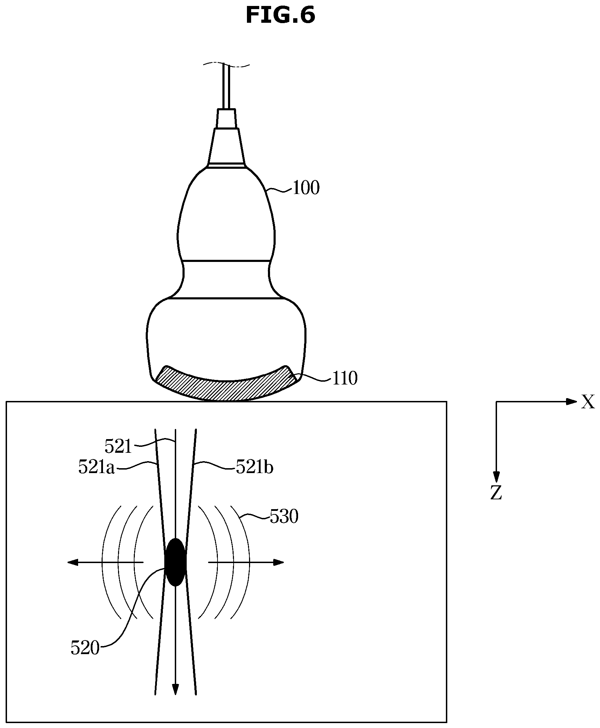

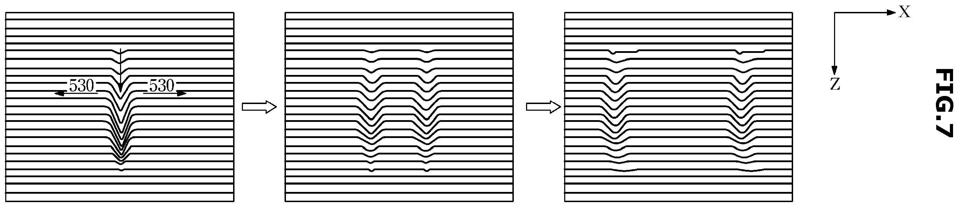

FIG. 6 illustrates induction of a shear wave by a push pulse. FIG. 7 illustrates propagation of a shear wave.

Referring to FIG. 6, the ultrasound probe 100 may transmit a push pulse 521 along a push line in a depth direction (Z direction) under the control of the ultrasound diagnostic apparatus 1. The push pulse may be irradiated to a focal point 520 in an ROI to induce displacement of the tissue and induce a shear wave 530. The push pulse is a focused beam having a relatively high focusing, and may have beam profiles 521a and 521b in a narrow width.

When the push pulse 521 is transmitted to the focal point 520 in the ROI, the shear wave 530 may be induced. That is, when a force is applied to a tissue of the focal point 520 in the depth direction (Z direction) by the push pulse 521, the tissue of the focal point 520 moves in the depth direction (Z direction). The distance moved by the tissue in the depth direction (Z direction) may be defined as a displacement. Since tissues of an object has a certain degree of elasticity, and adjacent tissues are organically connected, the movement of the tissue located at the focal point 520 also exerts influence on the adjacent tissues.

Referring to FIG. 7, the movement of the tissue located at the focal point 520 induces displacement of the adjacent tissues. The shear wave 530 may propagate in the X direction, which is a direction perpendicular to the depth direction (Z direction), due to the displacement of the tissues. The shear wave 530 propagates from the focal point 520 of the push pulse 521 to both sides. The shear wave 530 changes its velocity according to the vibrational characteristics of the medium. Therefore, the elasticity of a tissue may be obtained by estimating the shear wave velocity.

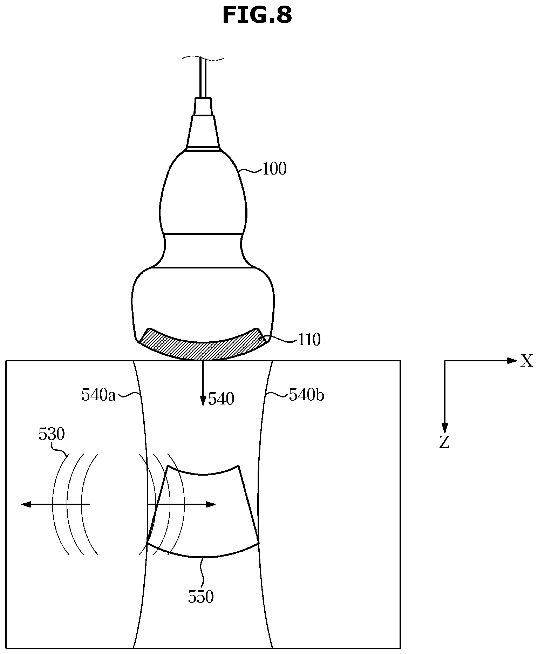

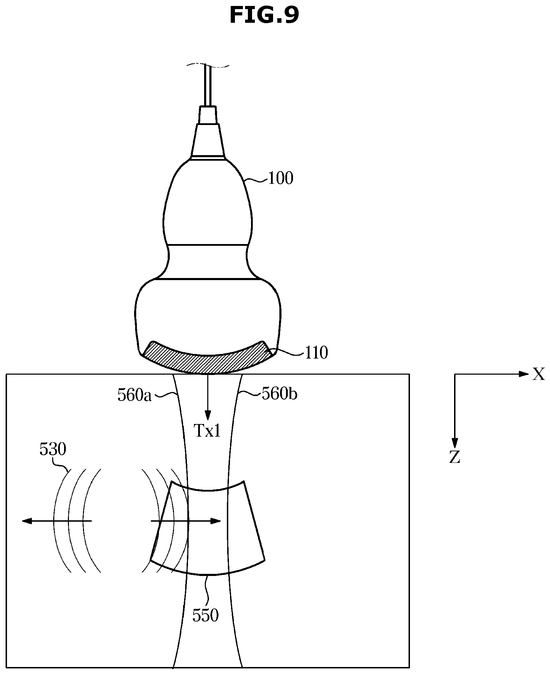

FIG. 8 illustrates an example of a method of detecting a shear wave. FIG. 9 illustrates transmission of a tracking pulse according to another example of a method of detecting a shear wave.

Referring to FIG. 8, the ultrasound probe 100 may transmit a tracking pulse 540 having wide beam profiles 540a and 540b to an ROI 550 in which the shear wave 530 propagates as a result of displacement of the tissues, and receive an ultrasound echo signal as a result of the tracking pulse 540 being reflected from the ROI 550. The ultrasound diagnostic apparatus 1 may detect the displacement of the tissues on the basis of the echo signal of the tracking pulse 540.

For example, the controller 300 may detect a displacement of a tissue by performing cross correlation on a first ultrasound image that is a reference ultrasound image and a second ultrasound image that is a shear wave photographing image. In other words, the controller 300 may compare the first ultrasound image (a reference ultrasound image) before application of the push pulse 521 with the second ultrasound image (a shear wave photographing image) after application of the push pulse 521, so that the degree to which the tissues are moved is detected.

In addition, the controller 300 may acquire shear wave photographing images at a high frame rate, and compare successive shear wave photographing image frames, so that displacement of the tissues are detected.

However, when measuring the elasticity of the tissue using the shear wave, the propagation of the shear wave needs to be accurately observed to accurately obtain the elasticity. As shown in FIG. 8, when the beam profiles 540a and 540b of the tracking pulse 540 are wide , the observing area is caused to be wide, and thus uniform observation of the ROI 550 is performable, but the observation performance (e.g., signal to noise ratio (SNR)) drops. The tracking pulse 540 shown in FIG. 8 is defined as a widely focused transmission beam (a widely focused transmission Tx beam).

In addition, when using the tracking pulse 540 having wide beam profiles 540a and 540b, the elasticity may not be accurately measured in an environment where reverberation exists. That is, since the transmission beam having a large width may include a lot of disturbances, the accuracy of the elasticity measurement may be reduced.

As shown in FIG. 9, when a tracking pulse Tx1 having narrow beam profiles 560a and 560b is used, a considerably high SNR may be obtained. The tracking pulse Tx1 shown in FIG. 9 may be defined as a tightly focused transmission beam (a tightly focused Tx beam). Using a narrow transmission beam may increase the accuracy of elasticity measurements even in an environment having reverberation. However, the tracking pulse Tx1 having narrow beam profiles 560a and 560b causes the observing area to be narrowed. Therefore, in order to widen the observing area of a tracking pulse Tx1, an interleaving or interrogation scheme is used.

A tightly focused tracking pulse Tx1 is transmitted into the ROI. The position of a focal point to which the tracking pulse Tx1 is transmitted may be adjusted on the basis of the ROI. That is, the focal point of the tracking pulse Tx1 may be moved into the ROI in response to movement of the ROI. At least one of the depth (Z direction) and the position in transverse direction (X direction) of the focal point of the tracking pulse Tx1 may be adjusted.

The beam profiles 560a and 560b of the tightly focused tracking pulse Tx1 are set to be smaller than the width of the ROI. That is, as shown in FIG. 9, the beam width of the tracking pulse Tx1 in the X direction at the focal point is set to be smaller than the width of the ROI.

FIG. 10 illustrates a method of transmitting a plurality of tracking pulses to widen an observing area.

Referring to the left side drawing of FIG. 10, in order to widen the observing area of the tracking pulse Tx1 having narrow beam profiles 560a and 560b, a plurality of tracking pulses Tx1, Tx2, Tx3, and Tx4 may be sequentially transmitted to a plurality of positions and/or focal points in the ROI. That is, after one push pulse 520 is transmitted along a push line 521, a plurality of tracking pulses Tx1, Tx2, Tx3, and Tx4 may be sequentially transmitted along respective transmission scan lines.

Before the plurality of tracking pulses Tx1, Tx2, Tx3, and Tx4 are transmitted to the ROI, the position of the focal point for each of the plurality of tracking pulses Tx1, Tx2, Tx3, and Tx4 is adjusted to be included in the ROI. In addition, the position of each focal point for each of the plurality of tracking pulses Tx1, Tx2, Tx3, and Tx4 may be adjusted such that the focal point is moved into the ROI according to movement of the ROI. Since the position of the focal point for each of the plurality of tracking pulses Tx1, Tx2, Tx3, and Tx4 is adjusted in response to movement of the ROI, the accuracy of the shear wave observation may be improved.

The controller 300 of the ultrasound diagnostic apparatus 1 performs sampling on echo signals of each of the plurality of tracking pulses Tx1, Tx2, Tx3, and Tx4. In addition, interpolation may be performed to increase the sampling rate. Such a shear wave observation method is referred to as an interleaving method because sampling is interleaved in time.

On the other hand, as the number of times of interleaving increases, the observation area becomes wider, and errors in estimating the shear wave velocity and the elasticity may decrease. In FIG. 10, four times of interleaving are illustrated as being performed. That is, FIG. 10 illustrates transmission of four tracking pulses.

In addition, referring to the right-side drawing of FIG. 10, a plurality of push pulses 520 may be sequentially transmitted along the push line 521. The plurality of push pulses 520 may be transmitted to the same focal point or to positions at different depths on the push line 521. In addition, the plurality of tracking pulses Tx1, Tx2, Tx3, and Tx4 may be sequentially transmitted to correspond to the respective push pulses 520. In other words, the ultrasound diagnostic apparatus 1 may transmit a first tracking pulse and transmit a first tracking pulse Tx1 to observe a shear wave, transmit a second push pulse and transmit a second tracking pulse Tx2 to observe a shear wave, transmit a third push pulse and transmit a third tracking pulse Tx3 to observe a shear wave, and then transmit a fourth push pulse and transmit a fourth tracking pulse Tx4 to observe a shear wave. Such a shear wave observation method is referred to as an interrogation method.

As described above, by sequentially transmitting the plurality of tracking pulses Tx1, Tx2, Tx3, and Tx4 to a plurality of points in the ROI, the observable area may be widened even using a tracking pulse having narrow beam profiles.

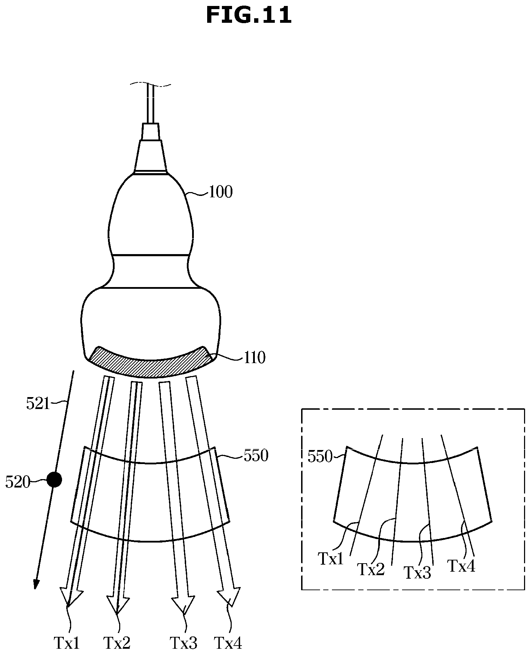

FIG. 11 illustrates radial transmission of a plurality of tracking pulses to suit a region of interest.

Referring to FIG. 11, the controller 300 of the ultrasound diagnostic apparatus 1 may set an ROI 550 in a radial shape, a fan shape, or a trapezoidal shape. The controller 300 may control the ultrasound probe 100 to radially transmit the plurality of tracking pulses Tx1, Tx2, Tx3, and Tx4 to correspond to the ROI 550 that is radially formed. That is, the controller 300 may control the transducer array 110 to radially transmit the plurality of tracking pulses Tx1, Tx2, Tx3, and Tx4.

When the ultrasound probe 100 is a convex type, the transducer array 110 may be formed in a curved surface. Therefore, with the convex type transducer array 110, the plurality of tracking pulses Tx1, Tx2, Tx3, and Tx4 may be transmitted in a radial shape. The controller 300 may selectively activate the transducer elements included in the transducer array 110. The controller 300 may activate different transducer elements to transmit a plurality of tracking pulses Tx1, Tx2, Tx3, and Tx4.

The ROI 550 may be set to have various sizes or widths. The user may set the ROI 550 using the control panel 260. Meanwhile, as the depth to which the ultrasound is irradiated increases, the size or width of the ROI 550 may be set larger. The controller 300 may set intervals between the plurality of tracking pulses Tx1, Tx2, Tx3, and Tx4 to correspond to the ROI 550 that is set. In addition, the controller 300 may set transmission angles of the plurality of tracking pulses Tx1, Tx2, Tx3, and Tx4 to correspond to the ROI 550.

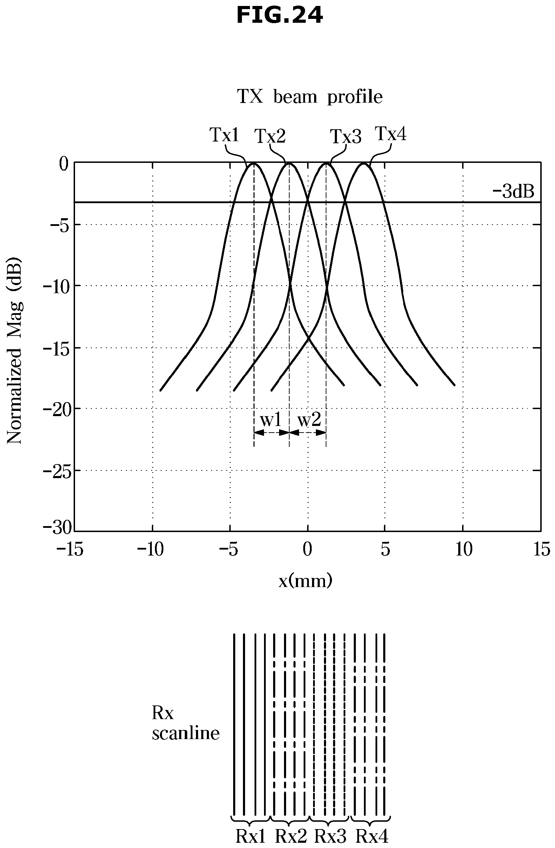

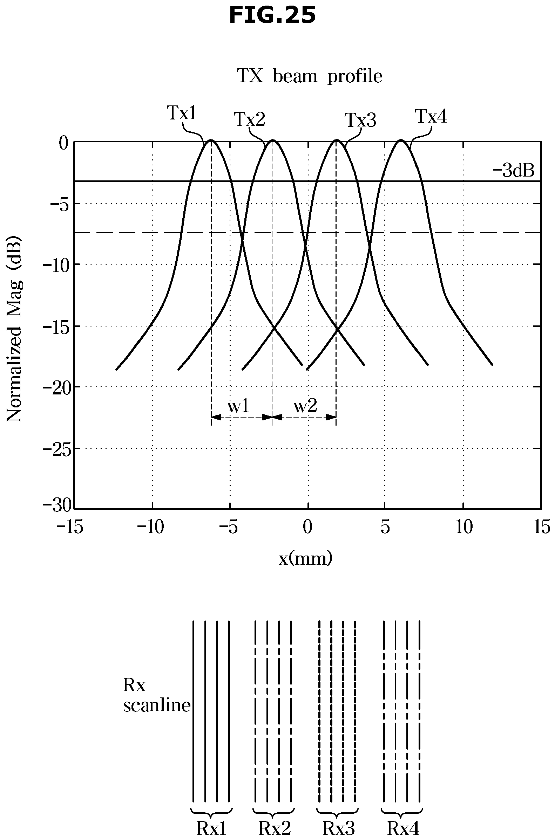

Setting the intervals between the plurality of tracking pulses Tx1, Tx2, Tx3, and Tx4 will be described with reference to FIGS. 24 and 25.

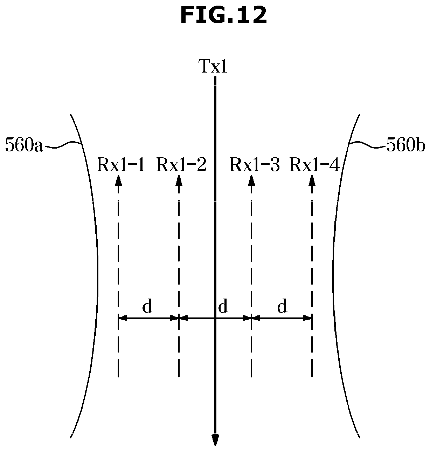

FIG. 12 illustrates multi-reception scan lines corresponding to a single tracking pulse.

Referring to FIG. 12, the controller 300 of the ultrasound diagnostic apparatus 1 may set multi reception scan lines Rx1-1, Rx1-2, Rx1-3, and Rx1-4 corresponding to a first tracking pulse Tx1. In FIG. 12, four reception scan lines Rx1-1, Rx1-2, Rx1-3, and Rx1-4 are illustrated. In general, the distance d between the multi-reception scan lines Rx1-1, Rx1-2, Rx1-3, and Rx1-4 is set constant.

The first tracking pulse Tx1 has beam profiles 560a and 560b with a predetermined width and transmits beams to positions corresponding to the four multi-reception scan lines Rx1-1, Rx1-2, Rx1-3, and Rx1-4. The transducer array 110 of the ultrasound probe 100 may receive ultrasound echo signals through the four reception scan lines Rx1-1, Rx1-2, Rx1-3, and Rx1-4. The controller 300 may appropriately delay and sum the ultrasound echo signals received through the four reception scan lines Rx1-1, Rx1-2, Rx1-3, and Rx1-4.

Beamforming using such multi-reception scan lines may reduce the ultrasound image acquisition time and increase the frame rate of the ultrasound image. Shear waves rapidly attenuate while propagating along the tissues, and thus travel a short distance for a short time. Therefore, by sampling the ultrasound echo signals received through the multi-reception scan lines, the shear wave may be easily observed.

On the other hand, increasing the number of reception scan lines may be considered as a method of increasing the sampling points. However, setting a large number of reception scan lines in response to a single tracking pulse may increase the width of the transmission beam. As described with reference to FIG. 8, when the width of the transmission beam is wide, a large amount of disturbance may be included, which may reduce the SNR and lower the accuracy of the elasticity measurement.

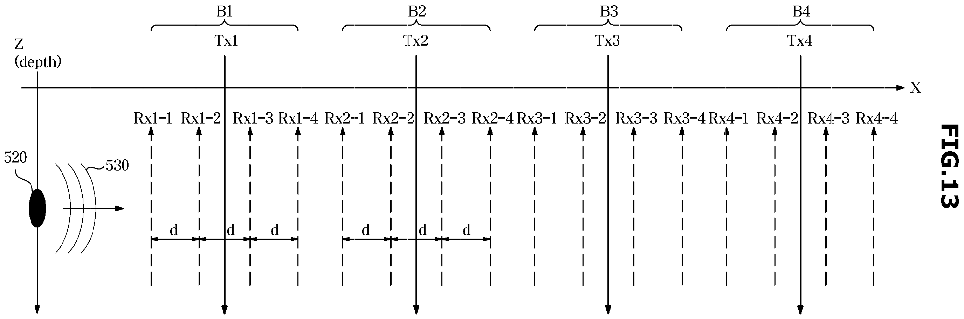

FIG. 13 illustrates a plurality of tracking pulses and a sequence of sets of multi-reception scan lines.

Referring to FIG. 13, a sequence of multi-reception scan lines corresponding to each of the four tracking pulses Tx1, Tx2, Tx3, and Tx4 is illustrated. The controller 300 may set sets B1, B2, B3, and B4 of the multi-reception scan lines to correspond to the plurality of respective tracking pulses. Specifically, the controller 300 may set a first set B1 from the first reception scan line to the fourth reception scan line Rx1-1, Rx1-2, Rx1-3, and Rx1-4 corresponding to the first tracking pulse Tx1. In addition, the controller 300 may set a second set B2 from the fifth reception scan line to the eighth reception scan line Rx2-1, Rx2-2, Rx2-3, and Rx2-4 corresponding to the second tracking pulse Tx2. Similarly, the controller 300 may set a third set B3 and a fourth set B4 of the multi-reception lines corresponding to the third and fourth tracking pulses Tx3 and Tx4, respectively.

With reference to the first reception scan line Rx1-1, as an example of the reception scan lines of the set B1 corresponding to the first tracking pulse Tx1, the first reception scan line Rx1-1 and the second reception scan Rx1-2 are disposed on the left side of the first tracking pulse Tx1, and the third reception scan line Rx1-3 and the fourth reception scan line Rx1-4 are disposed on the right side of the first tracking pulse Tx1.

In general, the distance d between the multi-reception scan lines Rx1-1, Rx1-2, Rx1-3, and Rx1-4 included in the same set is set constant. In addition, the distance d between adjacent sets of multi-reception scan lines is also set constant. For example, the distance d between the fourth reception scan line Rx1-4 and the fifth reception scan line Rx2-1 is set constant.

The ultrasound probe 100 may induce the shear wave 530 by transmitting a push pulse to the focal point 520 in the depth direction (Z direction). Since the shear wave 530 travels in the X direction, which is perpendicular to the depth direction (Z direction), the ultrasound probe 100 may transmit the first tracking pulses Tx1 to the fourth tracking pulse Tx4 along the X direction. When the shear wave is observed using the interleaving method, transmission beams by the plurality of tracking pulses Tx1, Tx2, Tx3 and Tx4 are sequentially transmitted to the positions of the plurality of reception scan lines Rx1-1 to Rx4-4. The ultrasound probe 100 may receive ultrasound echo signals as a result of the plurality of tracking pulses Tx1, Tx2, Tx3, and Tx4 being reflected along the sets B1, B2, B3, and B4 of multi reception scan lines.

The shear wave 530 sequentially reaches the positions of the plurality of reception scan lines Rx1-1 to Rx4-4. The controller 300 may detect displacement of a tissue corresponding to the position of the reception scan line on the basis of the ultrasound echo signals received along the plurality of reception scan lines Rx1-1 to Rx4-4. The controller 300 may detect displacement of tissues at a plurality of sampling points for each of the plurality of reception scan lines Rx1-1 to Rx4-4.

In addition, the controller 300 may estimate the arrival time of the shear wave 530 on each of the reception scan lines Rx1-1 to Rx4-4 on the basis of the displacement of the tissue. The controller 300 may estimate the velocity of the shear wave on the basis of the distance between the reception scan lines and a difference between the arrival times of the shear wave on the reception scan lines.

For example, the controller 300 may detect displacement of a tissue at a plurality of sampling points of the first reception scan line Rx1-1 and estimate the arrival time of the shear wave 530. Similarly, the controller 300 may detect displacement of a tissue at a plurality of sampling points of the second reception scan line Rx1-2 and estimate the arrival time of the shear wave 530. Subsequently, the controller 300 may estimate the velocity of the shear wave 530 on the basis of the distance d between the first reception scan line Rx1-1 and the second reception scan line Rx1-2 and the arrival time of the shear wave on each of the first reception scan line Rx1-1 and the second reception scan line Rx1-2.

On the other hand, the distance d between the first reception scan line Rx1-1 and the second reception scan line Rx1-2 may refer to the distance d between a first sampling point of the first reception scan line Rx1-1 and a second sampling point of the second reception scan line Rx1-2 that are located at the same depth.

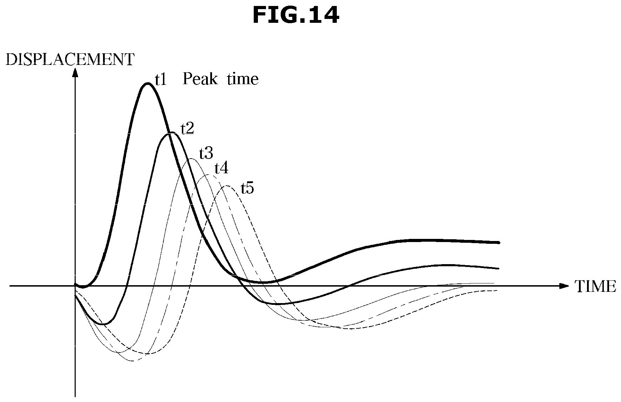

FIG. 14 illustrates the relationship between the displacement of a tissue and the arrival time of a shear wave.

Referring to FIG. 14, displacement of a tissue corresponding to the first sampling point of the first reception scan line Rx1-1 is maximum at time t1. Therefore, a first shear wave arrival time on the first sampling point of the first reception scan line Rx1-1 may be determined as t1. Similarly, displacement of a tissue corresponding to the second sampling point of the second reception scan line Rx1-2 is maximum at time t2. Therefore, a second shear wave arrival time on the second sampling point of the second reception scan line Rx1-2 may be determined as t2.

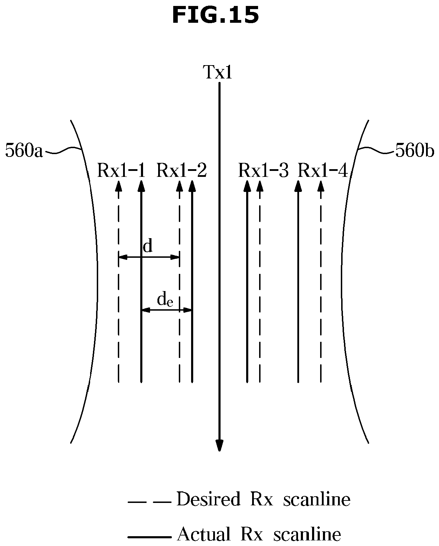

FIG. 15 illustrates the positional error of multi-reception scan lines.

Referring to FIG. 15, it can be seen that there is a difference between the ideal position and an actual position of the multi reception scan lines (the first reception scan line Rx1-1 to the fourth reception scan line Rx1-4) corresponding to the first tracking pulse Tx1.

As described above, the controller 300 arranges the first reception scan line Rx1-1 to the fourth reception scan line Rx1-4 on both sides of the first tracking pulse Tx1, and sets the distances d between the first reception scan line Rx1-1 to the fourth reception scan line Rx1-4 to be constant. However, since the beam of the first tracking pulse Tx1 is tightly focused, a positional error occurs between the set multi-reception scan line (dotted line) and the actual multi-reception scan line (solid line).

Specifically, the actually generated multi-reception scan lines tends to cluster around the tracking pulse Tx1. The first reception scan line Rx1-1 and the second reception scan line Rx1-2 are generated at positions shifted to the right side from the set positions. The third reception scan line Rx1-3 and the fourth reception scan line Rx1-4 are generated at positions shifted to the left side from the set positions. Accordingly, an error occurs between the distance d between the set first reception scan line Rx1-1 and the set second reception scan line Rx1-2 and the distance de between the actual first reception scan line Rx1-1 and the actual second reception scan line Rx1-2. The positional error of the reception scan lines increases as being distant from the tracking pulse Tx1.

The related art estimates the velocity of a shear wave using the entire sampling points of the multi-reception scan lines without considering such a positional error of the reception scan lines. Therefore, the estimated shear wave velocity is caused to have an error.

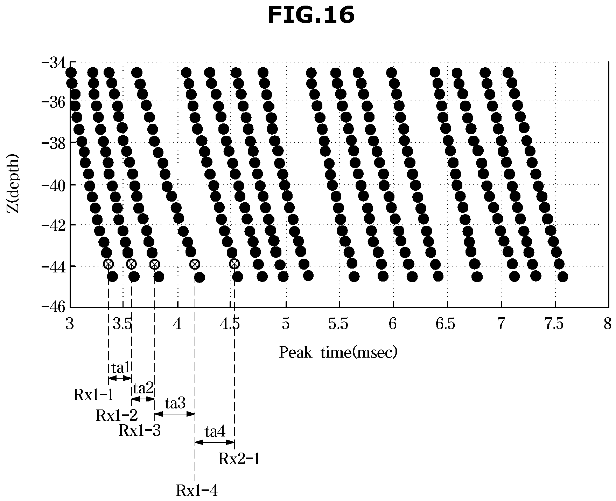

FIG. 16 illustrates the error of the shear wave arrival time on each of the multi-reception scan lines.

Referring to FIG. 16, a wave front graph showing the arrival times of a shear wave measured in a plurality of reception scan lines Rx1-1 to Rx4-4 is illustrated. FIG. 16 illustrates the points in time when a shear wave arrives at the respective sampling points of the plurality of reception scan lines Rx1-1 to Rx4-4. The numerical values shown in the wave front graph are illustrative purpose only, without being limited thereto.

As described above, when a tightly focused transmission beam is used, a positional error of reception scan lines may occur, and thus an error of shear wave arrival time on a plurality of reception scan lines Rx1-1 to Rx4-4 may also occur.

In FIG. 16, the shear wave arrival times on the first reception scan line Rx1-1 to the fifth reception scan line Rx2-1 are described. Referring to the sampling points located at a depth of -44 mm, the first shear wave arrival time on the first sampling point of the first receiving scan line Rx1-1 is approximately 3.4 ms, and the second shear wave arrival time on the second sampling point of the second reception scan line Rx1-2 is 3.6 ms, the third shear wave arrival time on the third sampling point of the third reception scan line Rx1-3 is 3.75 ms, the fourth shear wave arrival time on the fourth sampling point of the fourth reception scan line Rx1-4 is 4.1 ms, and the fifth shear wave arrival time on the fifth sampling point of the fifth reception scan line Rx2-1 is 4.5 ms.

In addition, a difference value $ta1$ between the first shear wave arrival time and the second shear wave arrival time is 0.2 ms, and a difference value $ta2$ between the second shear wave arrival time and the third shear wave arrival time is 0.15 ms, a difference value $ta3$ between the third shear wave arrival time and the fourth shear wave arrival time is 0.35 ms, and a difference value $ta4$ between the fourth shear wave arrival time and the fifth shear wave arrival time is 0.4 ms. As such, it can be seen that a positional error of the reception scan lines has occurred.

Various methods may be used to estimate the shear wave velocity. As an example, the velocity of the shear wave may be estimated on the basis of the distance between the first sampling point and the second sampling point and the difference value between the first shear wave arrival time and the second shear wave arrival time. As another example, the velocity of the shear wave may be estimated with respect to each of a plurality of sampling points using a plane equation or a wave equation, and adding up and averaging the velocities of the shear wave with respect to all sampling points.

However, the conventional shear wave velocity estimation methods reflecting the values of all the scan lines and sampling points have difficulty in removing the error of the shear wave velocities caused by the positional errors of the reception scan lines. Therefore, the reliability of the estimation result of the shear wave velocity is considerably low.

Hereinafter, a method of estimating the shear wave velocity that may remove the positional error of the reception scan line will be described. According to the disclosure, the shear wave velocity may be accurately obtained by selectively performing signal processing on multi-reception scan lines. Signal processing may refer to processing for ultrasound echo signals.

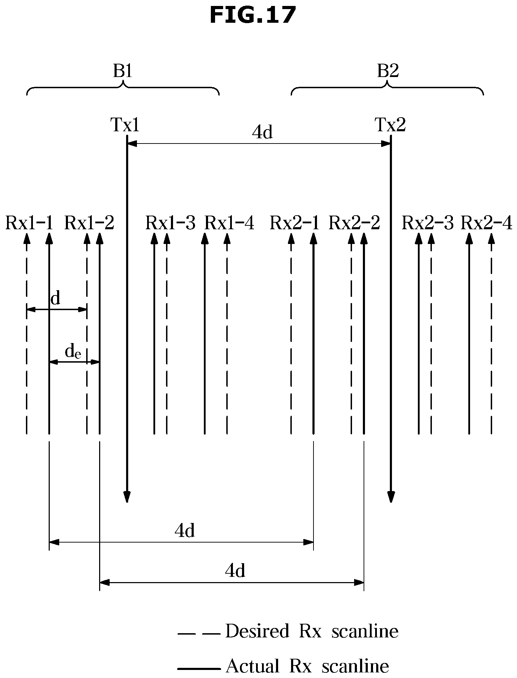

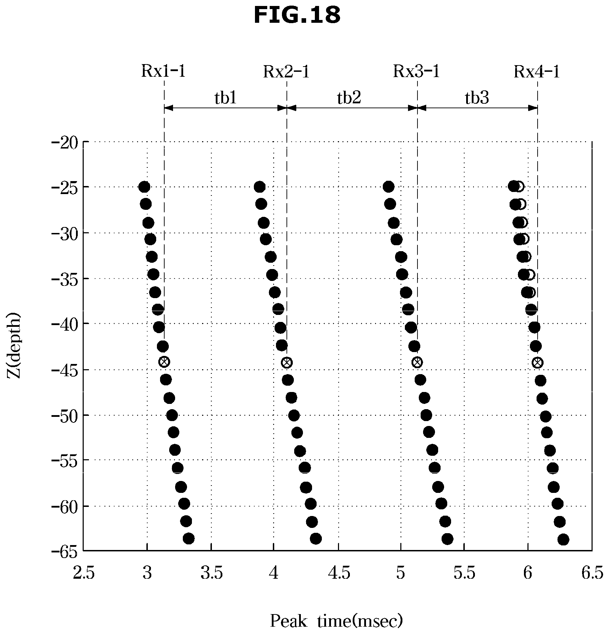

FIG. 17 illustrates reception scan lines positioned at the same relative location in the sets of the multi-reception scan lines. FIG. 18 illustrates the shear wave arrival times on reception scan lines positioned at the same relative location.

Referring to FIG. 17, when assuming that the plurality of tracking pulses Tx1, Tx2, Tx3, and Tx4 are transmitted at a constant interval 4d, the interval between the plurality of reception scan lines may also be set at a constant interval d. In addition, the positions of the actually generated reception scan lines may be different from the positions of the set reception scan lines as described above. Therefore, the distance de between the plurality of actually generated reception scan lines may be different from the distance d between the plurality of set reception scan lines.

However, the distance 4d between the reception scan lines located at the same relative position in each of the sets B1, B2, B3, and B4 of the multi reception scan line may be constant. For example, the distance between the first reception scan line Rx1-1 and the fifth reception scan Rx2-1 is 4d. Since the first reception scan line Rx1-1 and the fifth reception scan Rx2-1 have positional errors caused by the first tracking pulse Tx1 and the second tracking pulse Tx2, respectively, the first reception scan line Rx1-1 and the fifth reception scan Rx2-1 have the same relative position. In other words, since the first reception scan line Rx1-1 is a reception scan line arranged on the first position in the first set B1, and the fifth reception scan line Rx2-1 is a reception scan line arranged on the first position in the second set B2, the first reception scan line Rx1-1 and the second reception scan line Rx1-2 may be considered to have the same relative position. Similarly, the second reception scan line Rx1-2 and the sixth reception scan line Rx2-2 also exist on the same relative position.

FIG. 18 is a diagram illustrating reception scan lines located at same relative positions extracted from in the wave front graph of FIG. 16. That is, FIG. 18 illustrates the shear wave arrival times on the first reception scan line Rx1-1, the fifth reception scan line Rx2, the ninth reception scan line Rx3-1, and the thirteenth reception scan line Rx4-1, which are reception scan lines arranged at respective first positions in the sets B1, B2, B3, and B4 of the multi reception scan lines.

Referring to the sampling points located at a depth of -44 mm in FIG. 18, the first shear wave arrival time on the first sampling point of the first reception scan line Rx1-1 is approximately 3.2 ms, the fifth shear wave arrival time for the fifth sampling point of the fifth reception scan line Rx2-1 is 4.2 ms, the ninth shear wave arrival time for the ninth sampling point of the ninth reception scan line Rx3-1 is 5.2 ms, and the thirteenth shear wave arrival time for the thirteenth sampling point of the thirteenth reception scan line Rx4-1 is 6.2 ms.

In addition, a difference tb1 between the first shear wave arrival time and the fifth shear wave arrival time is 1 ms, and a difference tb2 between the fifth shear wave arrival time and the ninth shear wave arrival time is 1 ms, and a difference between the ninth shear wave arrival time and the thirteenth shear wave arrival time tb3 is 1ms. That is, it can be seen that the difference values between the shear wave arrival times on the reception scan lines at the same relative positions are the same.

As such, the ultrasound diagnostic apparatus 1 may remove the error of the shear wave velocity caused by the positional error of the reception scan line by estimating the shear wave velocity using the shear wave arrival times on the reception scan lines at the same relative position.

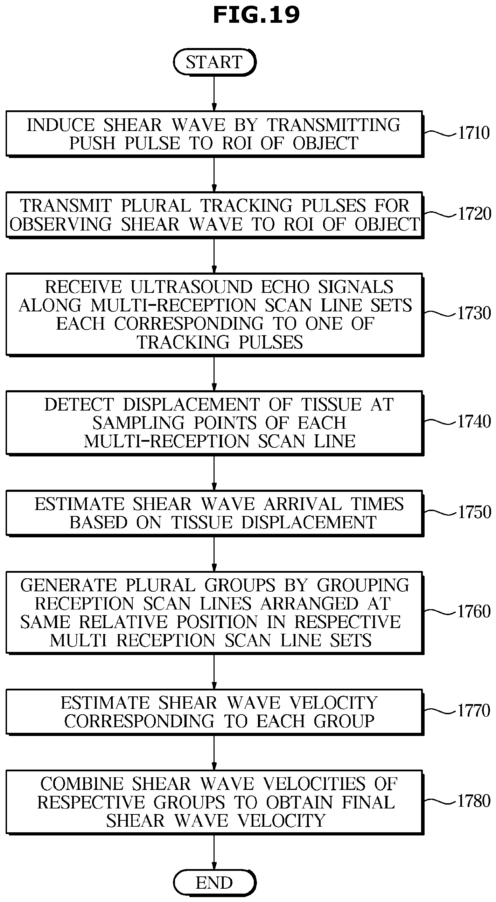

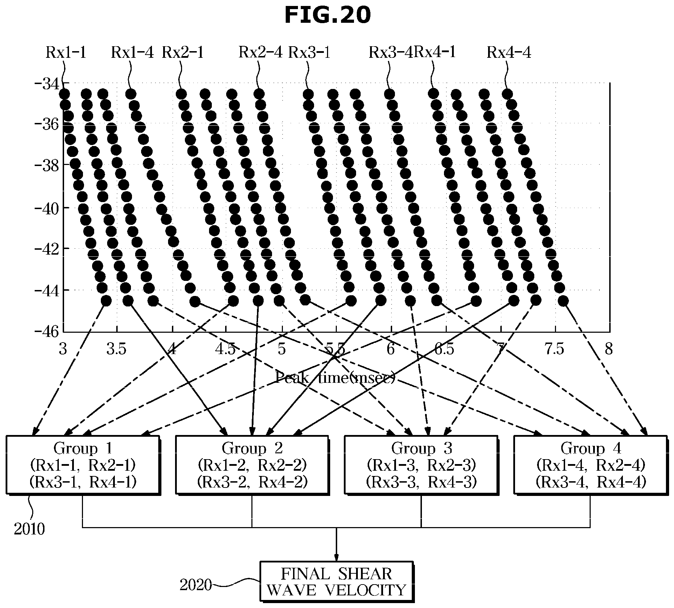

FIG. 19 is a flowchart showing a method of controlling an ultrasound diagnostic apparatus, which describes a method of estimating the shear wave velocity by grouping reception scan lines. FIG. 20 illustrates a wave front graph for describing the method of estimating the shear wave velocity shown in FIG. 19.

Referring to FIG. 19, the controller 300 of the ultrasound diagnostic apparatus 1 may control the ultrasound probe 100. The ultrasound probe 100 transmits a push pulse to an ROI of an object to induce a shear wave (1710). Thereafter, the ultrasound probe 100 transmits a plurality of tracking pulses Tx1, Tx2, Tx3, and Tx4 for observing the shear wave to the ROI of the object (1720). In this case, the intervals between the plurality of tracking pulses Tx1, Tx2, Tx3, and Tx4 may be adjusted on the basis of the ROI.

The ultrasound probe 100 receives ultrasound echo signals reflected from the ROI along sets B1, B2, B3, and B4 of multi-reception scan lines, each of the sets corresponding to a respective one of the plurality of tracking pulses (1730).

The controller 300 of the ultrasound diagnostic apparatus 1 may detect displacement of tissues at a plurality of sampling points of each of the multi-reception scan lines Rx1-1 to Rx4-4 (1740). In addition, the controller 300 may estimate the points in time when the shear wave arrives at the plurality of sampling points of each of the multi reception scan lines Rx1-1 to Rx4-4 (1750). FIG. 20 illustrates the point in time when the shear wave arrives at the plurality of sampling points of each of the reception scan lines Rx1-1 to Rx4-4.

The controller 300 may group reception scan lines arranged at the same relative position in the respective sets of the multi reception scan lines and generate a plurality of groups (1760 and 2010). Referring to FIG. 20, the first reception scan line Rx1-1, the fifth reception scan line Rx2-1, the ninth reception scan line Rx3-1, and the thirteenth reception scan line Rx4-1 are set to the first group. In addition, the second reception scan line Rx1-2, the sixth reception scan line Rx2-2, the tenth reception scan line Rx3-2, and the fourteenth reception scan line Rx4-2 are set to the second group.

The third reception scan line Rx1-3, the seventh reception scan line Rx2-3, the eleventh reception scan line Rx3-3, and the fifteenth reception scan line Rx4-3 are set to the third group, and the fourth reception scan line Rx1-4, the eighth reception scan line Rx2-4, the twelfth reception scan line Rx3-4, and the sixteenth reception scan line Rx4-4 are set to the fourth group.

The controller 300 may estimate a plurality of shear wave velocities each corresponding to one of the groups (1770). The controller 300 may estimate the first shear wave velocity for the first group, the second shear wave velocity for the second group, the third shear wave velocity for the third group, and the fourth shear wave velocity for the fourth group.

For example, in the case of the first group, the controller 300 may calculate the first shear wave velocity using the distance between the first sampling point of the first reception scan line Rx1-1 and the fifth sampling point of the fifth reception scan line Rx2-1 and the difference between the shear wave arrival times on the first sampling point and the fifth sampling point. The controller 300 may calculate the first shear wave velocity using the sampling points of the first reception scan line Rx1-1, the fifth reception scan line Rx2-1, the ninth reception scan line Rx3-1, and the thirteenth reception scan line Rx3-1 included in the first group.

On the other hand, the controller 300 may calculate the shear wave velocity with respect to each of the plurality of sampling points included in the first group using the plane equation or the wave equation, and calculate the first shear wave velocity by summing and averaging the shear wave velocities. The controller 300 may calculate the shear wave velocity for each group in various ways.

The controller 300 may combine the shear wave velocities of the respective groups to obtain the final shear wave velocity (1780 and 2020). In detail, the controller 300 may determine an average value of the plurality of shear wave velocities $v_i$ as the final shear wave velocity $v_{final}$, as shown in Equation 1 below.

[Equation 1]

$$v_{final} = \frac{1}{n} \sum_{i=1}^{n} v_i$$

In addition, the controller 30 may determine a weighted average value using the reliability measurement index (RMI) ($r_i$) for each of the shear wave velocities $v_i$ as the final shear wave velocity $v_{final}$ as shown in Equation 2 below.

[Equation 2]

$$v_{final} = \sum_{i=1}^{n} (v_i \times r_i) \div \sum_{i=1}^{n} r_i$$

On the other hand, the RMI with respect to the shear wave velocity may be obtained by combining the uniformity of the shear wave propagation, the magnitude of the shear wave displacement, the degree of correlation of the shear wave shape, and the like. In addition, the RMI may be calculated by various known methods.

For example, the controller 300 may calculate the first shear wave velocity for the first group in two different methods, and determine the RIM measure index for the first shear wave velocity on the basis of the proportions of the first shear wave velocities calculated by the two different methods. Specifically, the controller 300 may calculate a 1-1 shear wave velocity using a value obtained by averaging the distances from the focal point 520, to which the push pulse is transmitted, to the respective reception scan lines included in the first group and a value obtained by averaging the shear wave arrival times on the respective reception scan lines included in the first group. In addition, the controller 300 may calculate a 1-2 shear wave velocity using the first reception scan line Rx1-1 and the fifth reception scan line Rx2-1 as described above. The controller 300 may calculate a first shear wave velocity ratio SWV_ratio by dividing the difference between the 1-1 shear wave velocity and the 1-2 shear wave velocity by the 1-1 shear wave velocity. The controller 300 may determine an RMI of the first shear wave velocity on the basis of the first shear wave velocity ratio SWV_ratio.

On the other hand, when the shear wave velocity ratio SWV_ratio has a value greater than or equal to 0 and less than 0.5, the controller 300 may determine the value of the RMI to be 1. When the value of the shear wave velocity ratio SWV_ratio is greater than or equal to 0.5, the controller 300 may determine the value of the RMI by Equation 3 below.

[Equation 3]

RMI = -2 * SWV_ratio + 2

The controller 300 may calculate the elasticity of the tissue in the ROI on the basis of the final shear wave velocity and generate a shear wave elasticity image. The controller 300 may control the display 270 to output the shear wave elastic image. The shear wave elasticity image may be displayed to overlap or be registered with a reference ultrasound image. The reference ultrasound image may be a B-mode image. In addition, the controller 300 may control the display 270 to display elasticity, depth, and RMI.

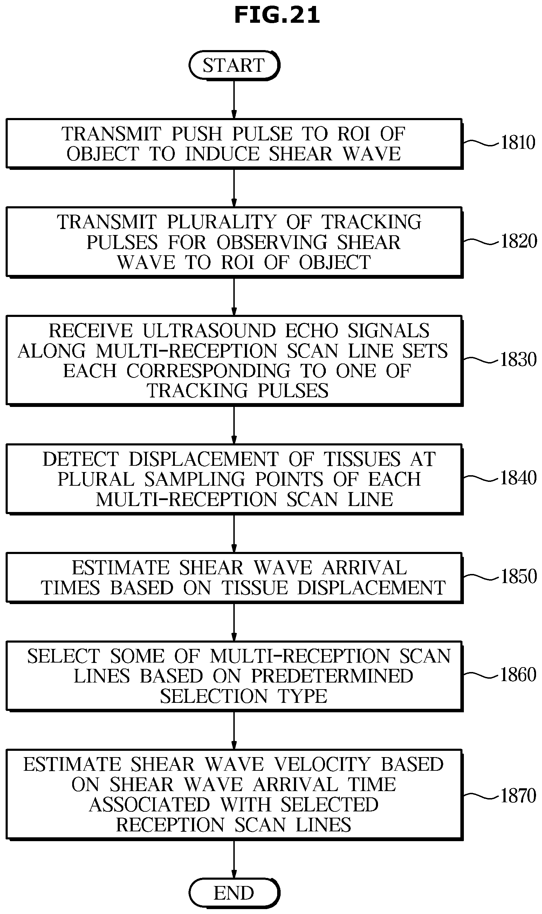

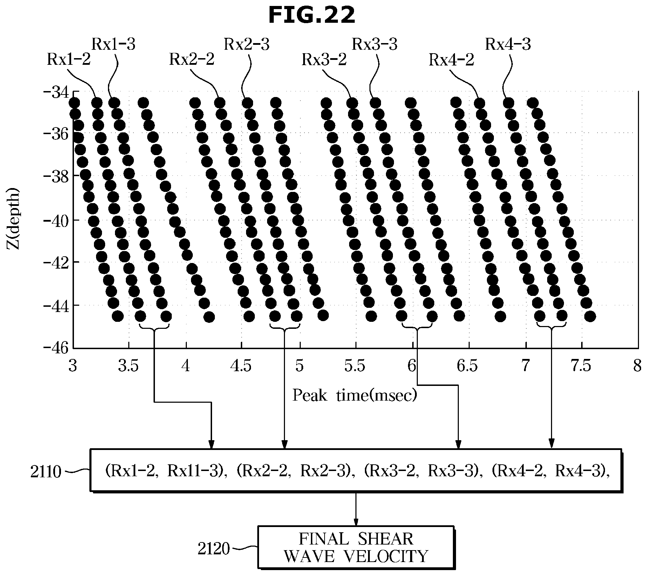

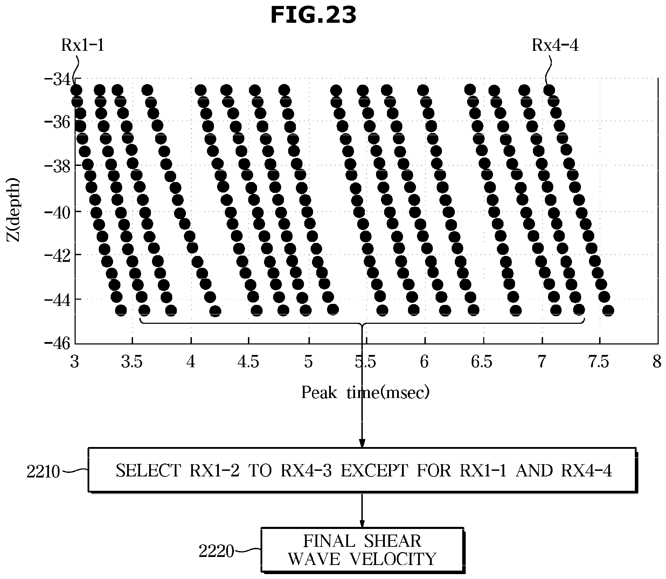

FIG. 21 is a flowchart showing a method of controlling an ultrasound diagnostic apparatus according to another embodiment, which describes a method of estimating the shear wave velocity by selecting some reception scan lines. FIGS. 22 and 23 show wave front graphs for describing the method of estimating the shear wave velocity shown in FIG. 21.

Referring to FIG. 21, the ultrasound probe 100 transmits a push pulse to an ROI of an object to induce a shear wave (1810), and transmits a plurality of tracking pulses Tx1, Tx2, Tx3, and Tx4 for observing the shear wave to the ROI of the object(1820). In this case, the intervals between the plurality of tracking pulses Tx1, Tx2, Tx3, and Tx4 may be adjusted on the basis of the ROI.

The ultrasound probe 100 receives ultrasound echo signals reflected from the ROI along sets B1, B2, B3, and B4 of multi-reception scan lines, each of the sets corresponding to a respective one of the plurality of tracking pulses (1830). The controller 300 may detect displacement of tissues at a plurality of sampling points of each of the multi-reception scan lines Rx1-1 to Rx4-4 (1840). In addition, the controller 300 may estimate the points in time when the shear wave arrives at the plurality of sampling points of each of the multi reception scan lines Rx1-1 to Rx4-4 (1850).

The controller 300 may select some reception scan lines of the multi-reception scan lines and estimate the shear wave velocity on the basis of ultrasound echo signals received along the selected reception scan lines. The controller 300 may select the reception scan lines on the basis of a predetermined selection type (1860, 2110, and 2210).

Referring to FIG. 22, as a first selection type, the controller 300 may select reception scan lines adjacent to the plurality of tracking pulses Tx1, Tx2, Tx3, and Tx4, in the sets B1, B2, B3, and B4 of the multi reception scan lines. Since each of the plurality of tracking pulses Tx1, Tx2, Tx3, and Tx4 forms a transmission beam, the controller 300 may select reception scan lines adjacent to the center of the transmission beam.

As shown in FIG. 22, the second reception scan line Rx1-2 and the third reception scan line Rx1-3 adjacent to the center of the transmission beam of the first tracking pulse Tx1 are selected, the fifth reception scan line Rx2-2 and the sixth reception scan line Rx2-3 adjacent to the center of the transmit beam of the second tracking pulse Tx2 are selected, the tenth scan line Rx3-2 and the eleventh reception scan line Rx3-3 adjacent to the center of the transmission beam of the third tracking pulse Tx3 are selected, and the fourteenth reception scan line Rx4-2 and the fifteenth reception scan line Rx4-3 adjacent to the center of the transmission beam of the fourth tracking pulse Tx4 are selected.

As described in FIG. 15, since the reception scan lines adjacent to the tracking pulse have a small positional error, the reception scan lines adjacent to the tracking pulse are selected, so that the error of the final shear wave velocity may be reduced.

Referring to FIG. 23, as a second selection type, the controller 300 may select reception scan lines except for a reception scan line having the minimum shear wave arrival time and a reception scan line having the maximum shear wave arrival time. On the wave front graph of FIG. 23, the reception scan line having the minimum shear wave arrival time is the first reception scan line Rx1-1, and the reception scan line having the maximum shear wave arrival time is the 16th reception scan line Rx4-4. Considering the positions of the reception scan lines are shifted to the center due to the transmission beam energy of the plurality of tracking pulses Tx1, Tx2, Tx3, and Tx4, the position of the outermost reception scan lines in the sequence of the plurality of reception scan lines may have the largest error.

In addition, as a third selection type (not shown), the controller 300 may select reception scan lines having a positional error smaller than a predetermined value.

The controller 300 may estimate the final shear wave velocity on the basis of the shear wave arrival times associated with the selected reception scan lines (1870, 2120, and 2220).

Meanwhile, the shear wave velocity estimation method described in FIGS. 21, 22, and 23 may be combined with the shear wave velocity estimation method described with reference to FIGS. 19 and 20. That is, the selected reception scan lines may be set as a plurality of groups, and the shear wave velocity may be estimated for each group.

FIGS. 24 and 25 illustrate the intervals between a plurality of tracking pulses.

Referring to FIG. 24, a beam profile graph of a plurality of tracking pulses Tx1, Tx2, Tx3, and Tx4 is shown. The controller 300 may set the intervals w1 and w2 between the plurality of tracking pulses Tx1, Tx2, Tx3, and Tx4 to be narrow such that flat areas (the area greater than -3 dB) of transmission beams of the plurality of tracking pulses Tx1, Tx2, Tx3, and Tx4. is used. The narrow setting of the intervals w1 and w2 between the plurality of tracking pulses Tx1, Tx2, Tx3, and Tx4 may be applied when a narrow ROI is observed.

In this case, since multi-reception scan lines Rx1, Rx2, Rx3, and Rx4 corresponding to each of the plurality of tracking pulses Tx1, Tx2, Tx3, and Tx4 are set to match the flat area (the area greater than -3 dB) of the transmission beam, the intervals between the reception scan lines may be set to be narrow. On the other hand, the intervals between the reception scan lines are set to be constant.

Referring to FIG. 25, the controller 300 may set the intervals between a plurality of tracking pulses Tx1, Tx2, Tx3, and Tx4 to be larger than a predetermined interval (for example, an interval of the transmission beam at -3dB) to use non-flat areas (the area smaller than -3 dB) of the plurality of tracking pulses Tx1, Tx2, Tx3, and Tx4. Even in this case, the beam widths of the plurality of tracking pulses Tx1, Tx2, Tx3, and Tx4 are tightly set. The wide setting of the intervals w1 and w2 between the plurality of tracking pulses Tx1, Tx2, Tx3, and Tx4 may be applied when observing a wide ROI. The intervals between the plurality of tracking pulses may be set without deviation from a range ROI.

For example, in order to observe a shear wave in an ROI larger than a predetermined magnitude using four tracking pulses Tx1, Tx2, Tx3 and Tx4, the intervals between the four tracking pulses need to be set large.

In this case, multi-reception scan lines Rx1, Rx2, Rx3, and Rx4 corresponding to each of the plurality of tracking pulses Tx1, Tx2, Tx3, and Tx4 are set to match the non-flat areas of the transmission beam (the area smaller than -3 dB), and thus the intervals between the reception scan lines may be set to be wide. Meanwhile, the intervals between the reception scan lines shown in FIG. 25 is larger than the intervals between the reception scan lines shown in FIG. 24.

Although not shown, the controller 300 may set the intervals between the plurality of tracking pulses Tx1, Tx2, Tx3, and Tx4 to be different from each other. The plurality of tracking pulses Tx1, Tx2, Tx3, Tx4 are transmitted to the ROI on the basis of a preset interval.

On the other hand, when the intervals between the plurality of tracking pulses are set to be larger than a predetermined interval as shown in FIG. 25, the benefit of the shear wave velocity estimation method described in FIGS. 19 to 23 may be provided. That is, since the shear wave velocity estimation method according to the disclosure selectively perform signal processing and/or data processing on multi-reception scan lines, the error of the shear wave velocity estimation may be reduced even when observing the shear wave for a wide ROI.

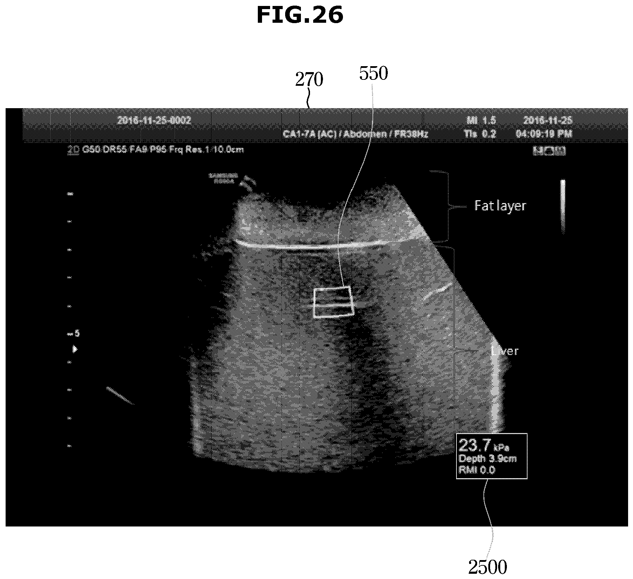

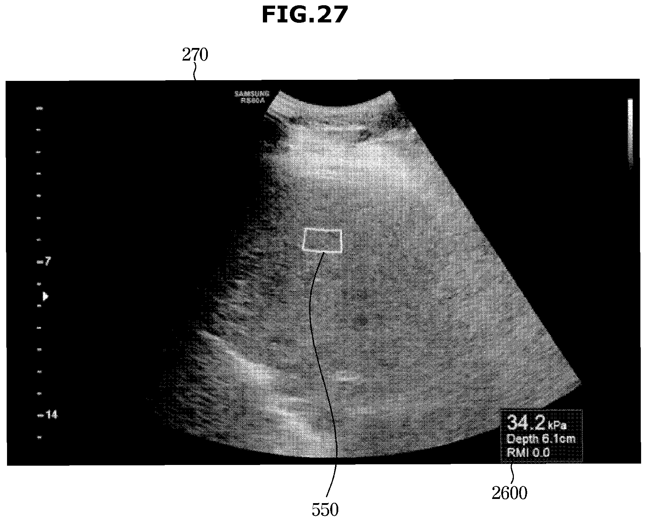

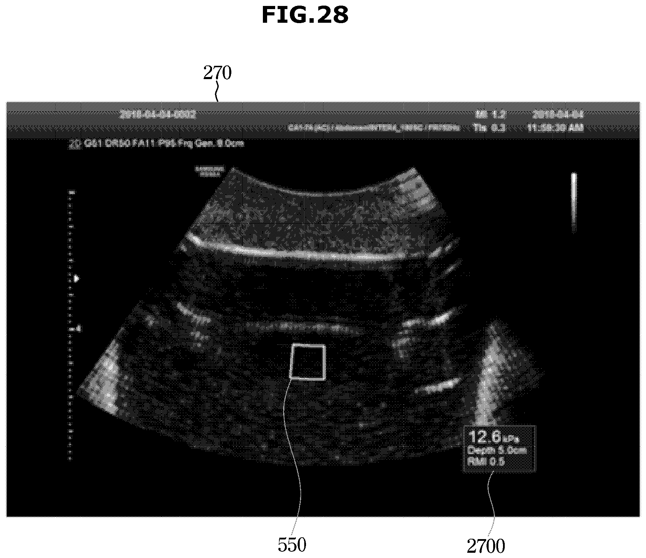

FIGS. 26 and 27 show the result of the elasticity measurement according to the related art. FIG. 28 shows the result of the elasticity measurement by the method of controlling the ultrasound diagnostic apparatus according to the embodiment.

FIGS. 26 to 28 illustrate ultrasound diagnosis images of a phantom including a liver and a fat layer. To generate an environment in which reverberation occurs, a phantom with a fat layer of 2 cm is used. On the other hand, the liver phantom has an elasticity value of 13kPa to 14kPa.

For comparison of the related art and the disclosure, an ROI 550 was set at a position in which the depth of the phantom is about 4 cm to 6 cm, and the elasticity of the ROI 550 was measured.

Referring to FIGS. 26 and 27, the related art failed to properly measure the elasticity value in an environment in which reverberation strongly occur. In FIG. 26, the elasticity value was measured at 23.7 kPa (see 2500). In FIG. 27, the elasticity value was measured at 34.2 kPa (see 2600). As such, the reliability of the elasticity value measured by the related art is very low.

On the other hand, referring to FIG. 28, it can be seen that the disclosure measured the elasticity value with a higher accuracy even in an environment in which reverberation occurs. That is, when the shear wave velocity estimation method according to the disclosure was used, the elasticity value was measured at 12.6 kPa (see 2700). The RMI value was calculated to be 0.5. As such, the elasticity value measured according to the disclosure is significantly close to the elasticity value of the phantom, and the reliability is very high.

As described above, according to the disclosed ultrasound diagnostic apparatus and the control method, the tracking pulse with a narrow beam width may improve the shear wave observation performance and may accurately measure the elasticity even in an environment in which reverberation occurs.

In addition, according to the disclosed ultrasound diagnostic apparatus and the control method, when the ROI is set wide, intervals between the tracking pulses for the shear wave observation are set to be wide, so that the elasticity may be accurately measured.

In addition, according to the disclosed ultrasound diagnostic apparatus and the control method, the shear wave velocity may be accurately obtained by selectively performing signal processing on the multi-reception scan line used to estimate the shear wave velocity.

Meanwhile, the disclosed embodiments may be embodied in the form of a recording medium storing instructions executable by a computer. The instructions may be stored in the form of program code and, when executed by a processor, may generate a program module to perform the operations of the disclosed embodiments. The recording medium may be embodied as a computer-readable recording medium.

The computer-readable recording medium includes all kinds of recording media in which instructions which may be decoded by a computer are stored, for example, a Read Only Memory (ROM), a Random-Access Memory (RAM), a magnetic tape, a magnetic disk, a flash memory, an optical data storage device, and the like.

As is apparent from the above, the ultrasound diagnostic apparatus and the method of controlling the same can improve the performance of shear wave observation and accurately measure the elasticity even in the presence of reverberation by narrowing the beam width of the tracking pulses.

The ultrasound diagnostic apparatus and the method of controlling the same can accurately measure the elasticity by setting the interval of tracking pulses for shear wave observation to be wide when a region of interest (ROI) is set wide.

In addition, the ultrasound diagnostic apparatus and the method of controlling the same can accurately measure the elasticity by selectively performing signal processing on multiple-reception scan lines used to estimate the shear wave velocity.

Although embodiments of the disclosure have been described for illustrative purposes, those skilled in the art will appreciate that various modifications, additions and substitutions are possible, as far as they fall within the scope of the invention, which is defined by the appended claims.

Therefore, embodiments of the disclosure have not been described for limiting purposes.

**Claims**

1. A method of controlling an ultrasound diagnostic apparatus, the method comprising:

    transmitting (1710; 1810) a push pulse (521) to a region of interest, ROI (550) of a target object to induce a shear wave (530);
    adjusting a position of a focal point (520) to which a plurality of tracking pulses (Tx1, Tx2, Tx3, Tx4) are transmitted, based on a position of the ROI;
    transmitting (1720; 1820) the plurality of tracking pulses to the ROI;
    setting sets (B1, B2, B3, B4) of multi-reception scan lines (Rx1-1 to Rx4-4), each set corresponding to a respective one of the plurality of tracking pulses;
    receiving (1730; 1830) ultrasound echo signals reflected from the ROI in response to the plurality of tracking pulses, along the multi-reception scan lines;
    estimating (1780; 1870) a velocity of the shear wave associated with the ROI based on the ultrasound echo signals received along the multi-reception scan lines;
    generating a shear wave elasticity image based on the velocity of the shear wave; and
    outputting the shear wave elasticity image on a display (280);
    **characterized in that** the estimating (1780; 1870) of the velocity of the shear wave includes at least one of:

    grouping (1760) reception scan lines positioned at a same relative position in each of the sets of the multi-reception scan lines to generate a plurality of groups, estimating (1770) a plurality of velocities of the shear wave each corresponding to a respective one of the plurality of groups, and determining (1780) a final velocity of the shear wave based on the plurality of the velocities of the shear wave; and
    selecting (1860) on the basis of a predetermined selection type some reception scan lines from the multi-reception scan lines and estimating (1870) the velocity of the shear wave based on ultrasound echo signals received along the selected some reception scan lines.

2. The method of claim 1, further comprising setting the ROI in a radial form,
    wherein the transmitting (1720; 1820) of the plurality of tracking pulses includes radially transmitting the plurality of tracking pulses to the ROI in the radial from.

3. The method of claim 1, wherein the adjusting of the position of the focal point includes moving the focal point into the ROI in response to movement of the ROI.

4. The method of claim 1, wherein the determining (1780) of the final velocity of the shear wave includes determining an average value of the plurality of velocities of the shear wave or a weighted average value obtained using a reliability measurement index, RMI, on each of the plurality of velocities of the shear wave as the final shear wave.

5. The method of claim 1, wherein the selecting (1860) of the reception scan lines includes selecting reception scan lines adjacent to each of the plurality of tracking pulses from the sets of the multi-reception scan lines.

6. The method of claim 1, wherein the selecting (1860) of the reception scan lines includes selecting reception scan lines having a positional error smaller than a predetermined value.

7. The method of claim 1, wherein the estimating (1780; 1870) of the velocity of the shear wave further includes estimating an arrival time of the shear wave on each of the multi-reception scan lines,
    wherein the selecting (1860) of the reception scan lines includes selecting reception scan lines except for a reception scan line in which the shear wave has a minimum arrival time and a reception scan line in which the shear wave has a maximum arrival time.

8. The method of claim 1, wherein the estimating (1780; 1870) of the velocity of the shear wave includes:

    detecting (1740; 1840) a displacement of a tissue at a plurality of sampling points of each of the multi-reception scan lines;
    estimating (1750; 1850) an arrival time of the shear wave on each of the multi-reception scan lines based on the displacement of the tissue; and
    estimating the velocity of the shear wave based on a distance between the grouped and/or selected reception scan lines and a difference between the arrival times of the shear wave on the grouped and/or selected reception scan lines.

9. An ultrasound diagnosis apparatus (1) comprising:

an ultrasound probe (100) configured to transmit a push pulse (521) to a region of interest, ROI (550) of a target object, transmit a plurality of tracking pulses (Txl, Tx2, Tx3, Tx4) to the ROI for observing a shear wave (530) that is induced by the push pulse, and receive ultrasound echo signals reflected from the ROI in response to the plurality of tracking pluses;

a controller (300) configured to adjust a position of a focal point (520) to which the plurality of tracking pulses are transmitted, on the basis of a position of the ROI, estimate a velocity of the shear wave associated with the ROI based on the ultrasound echo signals, generate a shear wave elasticity image based on the velocity of the shear wave; and

a display (280) on which the shear wave elasticity image is output;

wherein the controller (300) is configured to arrange sets (B1, B2, B3, B4) of multi-reception scan lines (Rx1-1 to Rx4-4), each set corresponding to a respective one of the plurality of tracking pulses;

**characterized in that** the controller (300) is configured to perform at least one of the following:

group reception scan lines positioned at a same relative position in each of the sets of the multi-reception scan lines to generate a plurality of groups, estimate a plurality of velocities of the shear wave each corresponding to a respective one of the plurality of groups, and determine a final velocity of the shear wave based on the plurality of the velocities of the shear wave; and

select on the basis of a predetermined selection type some reception scan lines from the multi-reception scan lines and estimate the velocity of the shear wave based on ultrasound echo signals received along the selected some reception scan lines.

10. The ultrasound diagnostic apparatus of claim 9, wherein the controller (300) sets the ROI in a radial form, and controls the ultrasound probe (100) to radially transmitting the plurality of tracking pulses to the ROI in the radial from.

**Patentansprüche**

1. Verfahren zur Steuerung einer Ultraschalldiagnosevorrichtung, wobei das Verfahren Folgendes umfasst:

Übertragen (1710; 1810) eines Push-Impulses (521) an einen interessierenden Bereich (ROI, Region Of Interest) (550) eines Zielobjekts, um eine Scherwelle (530) zu induzieren;

Anpassen einer Position eines Brennpunkts (520), an den eine Vielzahl von Nachverfolgungsimpulsen (Txl, Tx2, Tx3, Tx4) übertragen wird, basierend auf einer Position des ROI;

Übertragen (1720; 1820) der Vielzahl von Nachverfolgungsimpulsen an den ROI;

Einstellen von Sätzen (B1, B2, B3, B4) von Mehrfachempfangsabtastzeilen (Rx1-1 bis Rx4-4), wobei jeder Satz einem jeweiligen aus der Vielzahl von Nachverfolgungsimpulsen entspricht, Empfangen (1730; 1830) von Ultraschallechosignalen entlang den Mehrfachempfangsabtastzeilen, wobei die Ultraschallechosignale als Reaktion auf die Vielzahl von Nachverfolgungsimpulsen von der ROI reflektiert werden;

Schätzen (1780; 1870) einer Geschwindigkeit der Scherwelle, die dem ROI zugeordnet ist, basierend auf den entlang den Mehrfachempfangsabtastzeilen empfangenen Ultraschallechosignalen,

Erzeugen eines Scherwellenelastizitätsbildes basierend auf der Geschwindigkeit der Scherwelle; und

Ausgeben des Scherwellenelastizitätsbildes auf einer Anzeige (280);

**dadurch gekennzeichnet, dass** das Schätzen (1780; 1870) der Geschwindigkeit der Scherwelle mindestens eines von Folgendem umfasst:

Gruppieren (1760) von Empfangsabtastzeilen, die an einer gleichen relativen Position in jedem der Sätze der Mehrfachempfangsabtastzeilen positioniert sind, um eine Vielzahl von Gruppen zu erzeugen, Schätzen (1770) einer Vielzahl von Geschwindigkeiten der Scherwelle, die jeweils einer jeweiligen aus der Vielzahl von Gruppen entsprechen, und Bestimmen (1780) einer Endgeschwindigkeit der Scherwelle basierend auf der Vielzahl der Geschwindigkeiten der Scherwelle; und

Auswählen (1860) einiger Empfangsabtastzeilen aus den Mehrfachempfangsabtastzeilen auf der Grundlage eines vorbestimmten Auswahltyps, und Schätzen (1870) der Geschwindigkeit der Scherwelle basierend auf Ultraschallechosignalen, die entlang der ausgewählten einigen Empfangsabtastzeilen empfangen werden.

**2.** Verfahren nach Anspruch 1, das ferner das Einstellen des ROI in einer radialen Form umfasst, wobei das Übertragen (1720; 1820) der Vielzahl von Nachverfolgungsimpulsen das radiale Übertragen der Vielzahl von Nachverfolgungsimpulsen an den ROI in der radialen Form umfasst.

**3.** Verfahren nach Anspruch 1, wobei das Anpassen der Position des Brennpunkts das Bewegen des Brennpunkts in den ROI als Reaktion auf eine Bewegung des ROI umfasst.

**4.** Verfahren nach Anspruch 1, wobei das Bestimmen (1780) der Endgeschwindigkeit der Scherwelle das Bestimmen eines Durchschnittswerts der Vielzahl von Geschwindigkeiten der Scherwelle oder eines gewichteten Durchschnittswerts beinhaltet, der unter Verwendung eines Indikators für die Zuverlässigkeit der Messwerte (RMI, Reliability Measurement Index) für jede aus der Vielzahl von Geschwindigkeiten der Scherwelle als die letzte Scherwelle erhalten wird.

**5.** Verfahren nach Anspruch 1, wobei das Auswählen (1860) der Empfangsabtastzeilen das Auswählen von Empfangsabtastzeilen benachbart zu jedem aus der Vielzahl von Nachverfolgungsimpulsen aus den Sätzen der Mehrfachempfangsabtastzeilen umfasst.

**6.** Verfahren nach Anspruch 1, wobei das Auswählen (1860) der Empfangsabtastzeilen das Auswählen von Empfangsabtastzeilen mit einem Positionsfehler, der kleiner als ein vorbestimmter Wert ist, umfasst.

**7.** Verfahren nach Anspruch 1, wobei das Schätzen (1780; 1870) der Geschwindigkeit der Scherwelle ferner das Schätzen einer Ankunftszeit der Scherwelle auf jeder der Mehrfachempfangsabtastzeilen umfasst, wobei das Auswählen (1860) der Empfangsabtastzeilen das Auswählen von Empfangsabtastzeilen mit Ausnahme einer Empfangsabtastzeile, in der die Scherwelle eine minimale Ankunftszeit aufweist, und einer Empfangsabtastzeile, in der die Scherwelle eine maximale Ankunftszeit aufweist, beinhaltet.

**8.** Verfahren nach Anspruch 1, wobei das Schätzen (1780; 1870) der Geschwindigkeit der Scherwelle Folgendes beinhaltet:

Erfassen (1740; 1840) einer Verschiebung eines Gewebes an einer Vielzahl von Abtastpunkten jeder der Mehrfachempfangsabtastzeilen;
Schätzen (1750; 1850) einer Ankunftszeit der Scherwelle an jeder der Mehrfachempfangsabtastzeilen basierend auf der Verschiebung des Gewebes; und
Schätzen der Geschwindigkeit der Scherwelle basierend auf einem Abstand zwischen den gruppierten und/oder ausgewählten Empfangsabtastzeilen und einer Differenz zwischen den Ankunftszeiten der Scherwelle an den gruppierten und/oder ausgewählten Empfangsabtastzeilen.

**9.** Ultraschalldiagnosevorrichtung (1), die Folgendes umfasst:

eine Ultraschallsonde (100), die zu Folgendem konfiguriert ist: Übertragen eines Push-Impulses (521) an einen interessierenden Bereich (ROI, Region Of Interest) (550) eines Zielobjekts, Übertragen einer Vielzahl von Nachverfolgungsimpulsen (Txl, Tx2, Tx3, Tx4) an den ROI zum Beobachten einer Scherwelle (530), die durch den Push-Impuls induziert wird, und Empfangen von Ultraschallechosignalen, die als Reaktion auf die Vielzahl von Nachverfolgungsimpulsen von der ROI reflektiert werden;
eine Steuerung (300), die zu Folgendem konfiguriert ist: Anpassen einer Position eines Brennpunkts (520), an den die Vielzahl von Nachverfolgungsimpulsen übertragen wird, auf der Grundlage einer Position des ROI, Schätzen einer Geschwindigkeit der Scherwelle, die dem ROI zugeordnet ist, basierend auf den Ultraschallechosignalen, Erzeugen eines Scherwellenelastizitätsbildes basierend auf der Geschwindigkeit der Scherwelle; und
eine Anzeige (280), auf der das Scherwellenelastizitätsbild ausgegeben wird;
wobei die Steuerung (300) so konfiguriert ist, dass sie Sätze (B1, B2, B3, B4) von Mehrfachempfangsabtastzeilen (Rx1-1 bis Rx4-4) ausrichtet, wobei jeder Satz einem jeweiligen aus der Vielzahl von Nachverfolgungsimpulsen entspricht,
**dadurch gekennzeichnet, dass** die Steuerung (300) so konfiguriert ist, dass sie mindestens eines der Folgenden ausführt:

Gruppieren von Empfangsabtastzeilen, die an einer gleichen relativen Position in jedem der Sätze der Mehrfachempfangsabtastzeilen positioniert sind, um eine Vielzahl von Gruppen zu erzeugen, Schätzen einer Vielzahl von Geschwindigkeiten der Scherwelle, die jeweils einer jeweiligen aus der Vielzahl von Gruppen entsprechen, und Bestimmen einer Endgeschwindigkeit der Scherwelle basierend auf der Vielzahl der Geschwindigkeiten der Scherwelle; und

Auswählen einiger Empfangsabtastzeilen aus den Mehrfachempfangsabtastzeilen auf der Grundlage eines vorbestimmten Auswahltyps und Schätzen der Geschwindigkeit der Scherwelle basierend auf Ultraschallechosignalen, die entlang der ausgewählten einigen Empfangsabtastzeilen empfangen werden.

10. Ultraschalldiagnosevorrichtung nach Anspruch 9, wobei die Steuerung (300) den ROI in einer radialen Form einstellt und die Ultraschallsonde (100) steuert, um die Vielzahl von Verfolgungsimpulsen radial an den ROI in der radialen Form zu übertragen.

**Revendications**

1. Procédé de commande d'un appareil d'échographie diagnostique, le procédé comprenant :

l'émission (1710 ; 1810) d'une impulsion localisée (521) en direction d'une région d'intérêt (ROI, region of interest) (550) d'un objet cible pour induire une onde de cisaillement (530),
le réglage de la position d'un point de focalisation (520) vers lequel une pluralité d'impulsions de suivi (Txl, Tx2, Tx3, Tx4) sont émises, compte tenu de la position de la ROI,
l'émission (1720 ; 1820) de la pluralité d'impulsions de suivi en direction de la ROI,
l'établissement d'ensembles (B1, B2, B3, B4) de lignes de balayage multi-réception (Rx1-1 à Rx4-4), chaque ensemble correspondant à une impulsion respective de la pluralité d'impulsions de suivi,
la réception (1730 ; 1830) de signaux d'échos ultrasonores réfléchis par la ROI en réaction à la pluralité d'impulsions de suivi, le long des lignes de balayage multi-réception,
l'estimation (1780 ; 1870) de la vitesse de l'onde de cisaillement associée à la ROI compte tenu des signaux d'échos ultrasonores reçus le long des lignes de balayage multi-réception,
la génération d'un élastogramme à onde de cisaillement compte tenu de la vitesse de l'onde de cisaillement, et
la transmission de l'élastogramme à onde de cisaillement à un écran (280) ;
**caractérisé en ce que** l'estimation (1780 ; 1870) de la vitesse de l'onde de cisaillement comprend :

le groupement (1760) de lignes de balayage de réception disposées en une même position relative dans chacun des ensembles des lignes de balayage multi-réception de façon à générer une pluralité de groupes, l'estimation (1770) d'une pluralité de vitesses de l'onde de cisaillement, chacune correspondant à un groupe respectif de la pluralité de groupes, et la détermination (1780) de la vitesse finale de l'onde de cisaillement compte tenu de la pluralité de vitesses de l'onde de cisaillement ; et/ou
la sélection (1860), en fonction d'un type de sélection prédéterminé, de certaines lignes de balayage de réception parmi les lignes de balayage multi-réception, et l'estimation (1870) de la vitesse de l'onde de cisaillement compte tenu de signaux d'échos ultrasonores reçus le long desdites certaines lignes de balayage de réception sélectionnées.

2. Procédé selon la revendication 1, comprenant en outre l'établissement de la ROI sous une forme radiale, ladite émission (1720 ; 1820) de la pluralité d'impulsions de suivi comportant l'émission radiale de la pluralité d'impulsions de suivi en direction de la ROI sous la forme radiale.

3. Procédé selon la revendication 1, dans lequel le réglage de la position du point de focalisation comprend le déplacement du point de focalisation dans la ROI en réaction au déplacement de la ROI.

4. Procédé selon la revendication 1, dans lequel la détermination (1780) de la vitesse finale de l'onde de cisaillement comprend la détermination d'une valeur moyenne de la pluralité de vitesses de l'onde de cisaillement, ou d'une valeur moyenne pondérée obtenue au moyen d'un indice de fiabilité de la mesure (RMI, Reliability Measurement Index) sur chacune des vitesses de la pluralité de vitesses de l'onde de cisaillement, en tant qu'onde de cisaillement finale.

5. Procédé selon la revendication 1, dans lequel la sélection (1860) des lignes de balayage de réception comprend la sélection de lignes de balayage de réception adjacentes à chacune des impulsions de la pluralité d'impulsions de suivi parmi les ensembles des lignes de balayage multi-réception.

**6.** Procédé selon la revendication 1, dans lequel la sélection (1860) des lignes de balayage de réception comprend la sélection de lignes de balayage de réception dont l'erreur de position est inférieure à une valeur prédéterminée.

**7.** Procédé selon la revendication 1, dans lequel l'estimation (1780 ; 1870) de la vitesse de l'onde de cisaillement comprend en outre l'estimation de l'instant d'arrivée de l'onde de cisaillement sur chacune des lignes de balayage multi-réception,
dans lequel la sélection (1860) des lignes de balayage de réception comprend la sélection de lignes de balayage de réception à l'exception d'une ligne de balayage de réception dans laquelle l'onde de cisaillement a un instant d'arrivée minimal et d'une ligne de balayage de réception dans laquelle l'onde de cisaillement a un instant d'arrivée maximal.

**8.** Procédé selon la revendication 1, dans lequel l'estimation (1780 ; 1870) de la vitesse de l'onde de cisaillement comprend :

la détection (1740 ; 1840) d'un déplacement d'un tissu en une pluralité de points d'échantillonnage de chacune des lignes de balayage multi-réception,
l'estimation (1750 ; 1850) de l'instant d'arrivée de l'onde de cisaillement sur chacune des lignes de balayage multi-réception compte tenu du déplacement du tissu, et
l'estimation de la vitesse de l'onde de cisaillement compte tenu de la distance entre les lignes de balayage de réception groupées et/ou sélectionnées et d'une différence entre les instants d'arrivée de l'onde de cisaillement sur les lignes de balayage de réception groupées et/ou sélectionnées.

**9.** Appareil d'échographie diagnostique (1) comprenant :

une sonde échographique (100) conçue pour émettre une impulsion localisée (521) en direction d'une région d'intérêt (ROI, region of interest) (550) d'un objet cible, émettre une pluralité d'impulsions de suivi (Txl, Tx2, Tx3, Tx4) en direction de la ROI pour permettre l'observation d'une onde de cisaillement (530) induite par l'impulsion localisée, et recevoir des signaux d'échos ultrasonores réfléchis par la ROI en réaction à la pluralité d'impulsions de suivi,
un organe de commande (300) conçu pour régler la position d'un point de focalisation (520) vers lequel la pluralité d'impulsions de suivi sont émises, en fonction de la position de la ROI, estimer la vitesse de l'onde de cisaillement associée à la ROI compte tenu des signaux d'échos ultrasonores, générer un élastogramme à onde de cisaillement compte tenu de la vitesse de l'onde de cisaillement, et
un écran (280) auquel est transmis l'élastogramme à onde de cisaillement ;
ledit organe de commande (300) étant conçu pour agencer des ensembles (B1, B2, B3, B4) de lignes de balayage multi-réception (Rx1-1 à Rx4-4), chaque ensemble correspondant à une impulsion respective de la pluralité d'impulsions de suivi ;
**caractérisé en ce que** l'organe de commande (300) est conçu pour réaliser :

un groupement de lignes de balayage de réception disposées en une même position relative dans chacun des ensembles des lignes de balayage multi-réception de façon à générer une pluralité de groupes, une estimation d'une pluralité de vitesses de l'onde de cisaillement, chacune correspondant à un groupe respectif de la pluralité de groupes, et une détermination de la vitesse finale de l'onde de cisaillement compte tenu de la pluralité de vitesses de l'onde de cisaillement ; et/ou
une sélection, en fonction d'un type de sélection prédéterminé, de certaines lignes de balayage de réception parmi les lignes de balayage multi-réception, et une estimation de la vitesse de l'onde de cisaillement compte tenu de signaux d'échos ultrasonores reçus le long desdites certaines lignes de balayage de réception sélectionnées.

**10.** Appareil d'échographie diagnostique selon la revendication 9, dans lequel l'organe de commande (300) établit la ROI sous une forme radiale, et commande la sonde échographique (100) de façon qu'elle émette radialement la pluralité d'impulsions de suivi en direction de la ROI sous la forme radiale.

# FIG. 1

# FIG.2

# FIG.3

100

170                                    120

PROBE
CONTROLLER                         CABLE
                            171
                                                130
110              PROCESSOR
                                    CONNECTOR
TRANSDUCER
ARRAY
                            172

                 MEMORY

# FIG.4

START

INDUCE SHEAR WAVE BY TRANSMITTING PUSH PULSE TO ROI OF OBJECT — 410

TRANSMIT TRACKING PULSES FOR OBSERVING SHEAR WAVE TO ROI OF OBJECT, AND RECEIVE ULTRASOUND ECHO SIGNALS — 420

DETECT DISPLACEMENT OF TISSUE BY SHEAR WAVE AT PLURAL SAMPLING POINTS IN ROI — 430

ESTIMATE SHEAR WAVE ARRIVAL TIMES BASED ON DISPLACEMENT OF TISSUE — 440

ESTIMATE SHEAR WAVE VELOCITY BASED ON DISTANCE BETWEEN PLURALITY OF SAMPLING POINTS AND SHEAR WAVE ARRIVAL TIMES — 450

END

# FIG.5

# FIG.6

# FIG.7

# FIG.8

# FIG.9

# FIG.10

Tx1 Tx2 Tx3 Tx4

<Interleaving>

Tx1 Tx2 Tx3 Tx4

<Interrogation>

# FIG.11

# FIG.12

FIG.13

EP 3 711 678 B1

# FIG.14

DISPLACEMENT

t1  Peak  time

t2

t3
t4
t5

TIME

# FIG.15

— — Desired Rx scanline

—— Actual Rx scanline

# FIG.16

# FIG.17

— — Desired Rx scanline

——— Actual Rx scanline

**FIG.18**

# FIG.19

START

INDUCE SHEAR WAVE BY TRANSMITTING PUSH PULSE TO ROI OF OBJECT ~1710

TRANSMIT PLURAL TRACKING PULSES FOR OBSERVING SHEAR WAVE TO ROI OF OBJECT ~1720

RECEIVE ULTRASOUND ECHO SIGNALS ALONG MULTI-RECEPTION SCAN LINE SETS EACH CORRESPONDING TO ONE OF TRACKING PULSES ~1730

DETECT DISPLACEMENT OF TISSUE AT SAMPLING POINTS OF EACH MULTI-RECEPTION SCAN LINE ~1740

ESTIMATE SHEAR WAVE ARRIVAL TIMES BASED ON TISSUE DISPLACEMENT ~1750

GENERATE PLURAL GROUPS BY GROUPING RECEPTION SCAN LINES ARRANGED AT SAME RELATIVE POSITION IN RESPECTIVE MULTI RECEPTION SCAN LINE SETS ~1760

ESTIMATE SHEAR WAVE VELOCITY CORRESPONDING TO EACH GROUP ~1770

COMBINE SHEAR WAVE VELOCITIES OF RESPECTIVE GROUPS TO OBTAIN FINAL SHEAR WAVE VELOCITY ~1780

END

# FIG.20

# FIG.21

START

TRANSMIT PUSH PULSE TO ROI OF OBJECT TO INDUCE SHEAR WAVE — 1810

TRANSMIT PLURALITY OF TRACKING PULSES FOR OBSERVING SHEAR WAVE TO ROI OF OBJECT — 1820

RECEIVE ULTRASOUND ECHO SIGNALS ALONG MULTI-RECEPTION SCAN LINE SETS EACH CORRESPONDING TO ONE OF TRACKING PULSES — 1830

DETECT DISPLACEMENT OF TISSUES AT PLURAL SAMPLING POINTS OF EACH MULTI-RECEPTION SCAN LINE — 1840

ESTIMATE SHEAR WAVE ARRIVAL TIMES BASED ON TISSUE DISPLACEMENT — 1850

SELECT SOME OF MULTI-RECEPTION SCAN LINES BASED ON PREDETERMINED SELECTION TYPE — 1860

ESTIMATE SHEAR WAVE VELOCITY BASED ON SHEAR WAVE ARRIVAL TIME ASSOCIATED WITH SELECTED RECEPTION SCAN LINES — 1870

END

# FIG.22

**FIG.23**

# FIG.24

# FIG.25

TX beam profile

# FIG.26

270    550

2500

# FIG.27

270

550

2600

# FIG.28

270

550        2700

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2018060820 A1

### Non-patent literature cited in the description

- Two-dimensional shear-wave elastography on conventional ultrasound scanners with time-aligned sequential tracking (TAST) and comb-push ultrasound shear elastography (CUSE). **SONG PENGFEI et al.** IEEE TRANSACTIONS ON ULTRASONICS, FERROELECTRICS AND FREQUENCY CONTROL. IEEE, 01 February 2015, vol. 62

- **SONG PENGFEI et al.** Fast Shear Compounding Using Robust 2-D Shear Wave Speed Calculation and Multi-directional Filtering. *ULTRASOUND IN MEDICINE & BIOLOGY,* 01 June 2014, vol. 40 (6